# EUROPEAN PATENT SPECIFICATION

(11) **EP 4 323 046 B1**
(45) Date of publication and mention of the grant of the patent: **11.11.2026**
(21) Application number: 22723714.6
(22) Date of filing: 12.04.2022
(51) Int. Cl.: A61M 25/01, A61M 25/09, A61M 25/06

(54) **DEVICE FOR INSERTING A TUBE INTO A BODY CAVITY**
VORRICHTUNG ZUM EINFÜHREN EINES SCHLAUCHS IN EINE KÖRPERHÖHLE
DISPOSITIF POUR INSÉRER UN TUBE DANS UNE CAVITÉ CORPORELLE

(30) Priority: 13.04.2021 GB 202105228
(43) Date of publication of application: 21.02.2024
(73) Proprietor: Eazyiv Ltd, Stockbridge, SO20 8AD (GB)
(72) Inventor: BOWMAN, George Henry Peter, London, SW19 4QA (GB)
(74) Representative: Gillott-Jones, Nathan Philip
(86) International application number: PCT/GB2022/000040
(87) International publication number: WO 2022/219295

(56) References cited:
- EP-A2- 0 832 663
- WO-A1-2013/142386
- WO-A2-2008/005618
- WO-A2-98/19720
- US-A- 5 415 177

## Description

### Field of The Invention

This invention relates to the field of medical devices. In particular, though not exclusively, to devices, and the insertion of tubes such as needles, ports, cannulas, or catheters in uses such as intravascular catheterisation.

### Background

A cannula or catheter is a thin tube which can be inserted into a vein or body cavity to administer medication, drain off fluid, or insert a surgical instrument. The terms "cannula" and "catheter" are often used interchangeably. In this specification, "cannula" will mean the whole medical device including a catheter introducer needle and associated mechanism and "catheter" to refer to just the tube (typically plastic) that fits concentrically around the introducer needle and remains after withdrawal of the introducer needle part of the device. The catheter can then act as a conduit for e.g., introducing medication into, or taking blood samples from, a patient. In this specification, the terms "vein" and "venous" may be used to refer to veins or arteries. The term "intravascular catheterisation" is understood by the skilled addressee to cover both intravenous and intra-arterial catheterisation.

An estimated 67% of all hospital patients have a Peripheral Intravenous Cannula (PIVC) inserted making this the most frequent medical procedure performed in hospitals worldwide. 330 million cannulas (or "catheters" as they are known in the US) were used in the USA in 2014.

One third of adults and half of children have difficult venous access and a highly experienced user is therefore required to insert the PIVC. The venous access may be difficult for several reasons including the size of the blood vessel, its fragility, depth, and/or state of collapse, or tortuosity. Other factors include skin colour, chronic disease, and venous depletion, or lymphoedema. Furthermore, the presence of valves or junctions in veins may impede or prevent insertion of a catheter. These patients can endure multiple painful attempts at catheterisation, a delay in diagnosis, a delay in commencement of treatment, and potential escalation to central venous access.

A further issue in the developed nation is the increasing levels of obesity in the population. It is more difficult to introduce an intravenous catheter into an obese patient, and even superficial veins in such patients can become deep relative to the patient's skin.

The method of inserting a PIVC has remained largely unchanged since their invention in the 1880's. It is estimated that up to 40% of first attempts to catheterise fail. Multiple attempts at catheterisation increase the risks of phlebitis, thrombosis and catheter-related infection leading to premature device failure.

The insertion of an intravascular catheter in general is a skilled procedure, requiring care to find a blood vessel, and to introduce the cannula introducer needle and catheter though the proximal wall of the vessel and into the lumen. Practical issues with the use of catheters are discussed in Harty, E. Update in Anaesthesia p22 *et seq.* https://www.e-safe-anaesthesia.org/e_library/05/Peripheral_intravenous_cannulae_update_2011.pdf As described in this document, the insertion of needle into a vein is indicated visually to a user by a first "flashback" of blood in the needle. After withdrawal of the needle, a second flow of blood is seen in the catheter itself generally indicating that the catheter alone is in the vein.

Common causes of failure in the introduction of tubes into body cavities and especially the placement of PIVC include overpenetration and under-penetration. In the case of intravascular catheterisation, the first flashback described in in Harty, E *supra* alerts the user that the needle is in the lumen of the vein. The distance from the tip of the needle to the distal end of the catheter is in the region of 2mm - similar to the diameter of the vein. On viewing the flashback, the user must manipulate the catheter over the tip of the needle and advance the catheter while keeping the needle stationary. This usually requires both hands. It can be appreciated that if either the patient or the user moves, or if the manipulation is not skilled enough, the needle may over-penetrate - where the cannula needle and/or catheter passes unintentionally through a distal blood vessel wall, rather than remaining within the lumen of the blood vessel. Alternatively, the needle may underpenetrate - where the cannula needle and or catheter exits or fails to reach the lumen of the vein and is advanced through the tissue along the outside of the vein wall. Over-penetration and under-penetration are significant problems with conventional catheter designs which rely on the manual dexterity of a skilled user. Overpenetration and under-penetration may cause unnecessary damage to surrounding tissues and organs, as well as unpleasant bruising. Overpenetration and under-penetration may still result in a visually apparent "flashback" of blood with conventional cannulas which may mislead a user.

Commonly used cannulas for intravascular catheterisation are relatively simple and include the BD Venflon Pro Safety cannula, produced by Becton Dickinson Infusion Therapy AB, which includes features to avoid secondary needlestick injuries on removal of the needle from the catheter.

Another cannula is disclosed in US2008/0300574 (BELSON AMIR), which includes a metal guidewire arranged within a hollow introducer needle which supports a concentric catheter in conventional manner. In use, after the insertion of the needle into the lumen of a vein, the guidewire is manually extended from within the periphery of the needle along the lumen of the vein. After the guidewire has been extended from the needle, the catheter is advanced along the guidewire. When the catheter is fully advanced, extending along the lumen of the vein, the needle and guidewire are retracted and disposed of.

The Arrow QuickFlash Radial Artery catheterisation set, produced by Arrow International, Inc. is intended for one handed operation in intra-arterial catheterisation which is a less common procedure than intravenous catheterisation. The cannula includes a needle which is moveable within a polyurethane polymer catheter. An optional guide wire may be manually advanced along the lumen of an artery after needle insertion through the proximal artery wall to guide the catheter through the artery.

US2004/0116864 (BOUDREAUX) discloses a catheter introducer assembly having safety shielded needle before and after use, the assembly includes an elongate "blunt" which can be extended from the hollow needle to protect the end of the needle, but all of the operation is under the manual control of the user.

US5,702,367 (COVER/BECTON DICKINSON CO) discloses a cannula design intended to better control leakage, retraction speed and reuse. An insertion needle is spring-loaded for retraction into the device after catheter insertion. Needle retraction is manually triggered by a user. The invention seems to relate to controlling the retraction of the needle to improve the user experience of the device.

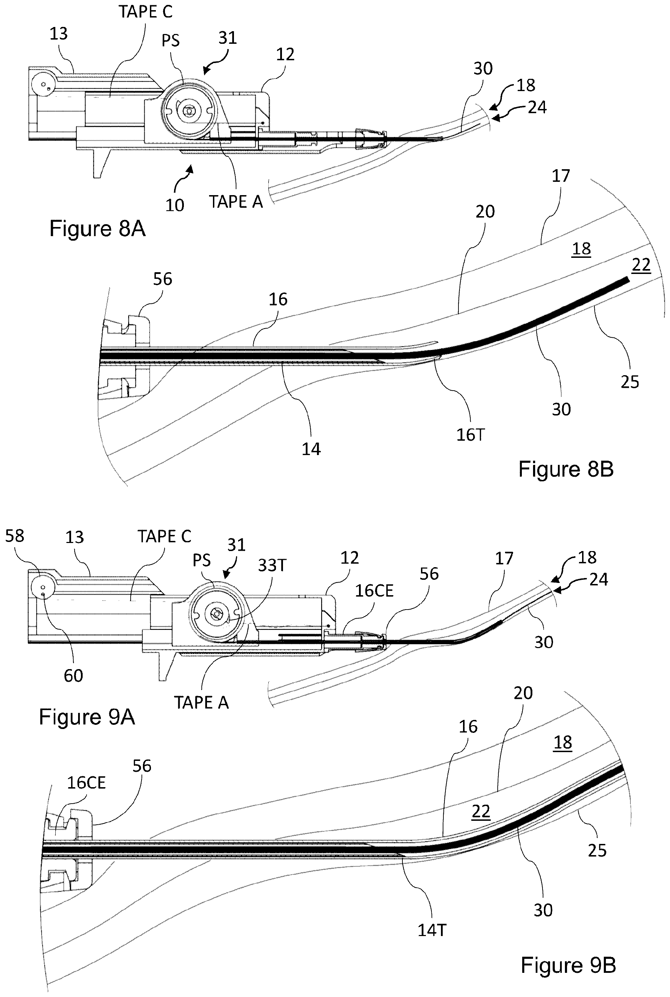

US5,330,432 (YOON) relates to a retractable safety penetrating instrument. The focus of the disclosure is on achieving automatic needle retraction soon after penetration of a body cavity to avoid the possibility of overpenetration. Several embodiments of the instrument are described which include one spring which distally biases the introducer needle and another stronger retraction spring which quickly retracts the needle on cavity penetration. The retracting spring is released by a trigger mechanism. This trigger mechanism may be difficult to manufacture such that it acts reliably and may tend to jolt unacceptably in use. In one embodiment, described in relation to Figure 8 of US5,330,432, a "safety probe" is arranged within the needle with both the "safety probe" and needle of the Figure 8 embodiment retracting soon after or upon cavity entry by a cannula. It is also noted that there is no balancing or linkage of the forces applied by the springs in the device of US5,330,432. Furthermore, the safety probe moves in step with the needle. From the references to "irrigation" in that embodiment it is apparent that the instrument is not intended for applications such as intravascular catheterisation.

US5,415,177 (ZADINI) discloses an intravascular catheterisation device with automatically actuated means for moving a guidewire distally when penetration of the blood vessel wall is sensed. Embodiments include pneumatic/vacuum or magnetic advancement of the guidewire. This provides a form of automatic advancement of the guidewire, but this seems to be relatively uncontrolled in nature, there being no fine control of the probe's position. In particular, US5,415,177 describes a guidewire placement device for intravascular catheters comprising a guidewire, a hollow needle and self-propelled means for moving said guidewire to an advanced position upon penetration of the wall of a blood vessel by the needle. The self-propelling means may be actuated manually or may include means for sensing penetration of a blood vessel by the needle and for automatically advancing the guidewire in response to such penetration.

US10,118,020 (AVNERI) relates to automatic advancement of a guidewire, in response to a detected physiological parameter such as blood pressure by a sensor. The sensor may be a pressure sensor, a conductivity sensor, a flow sensor, an ultrasonic sensor, a photoelectric sensor, or a resistance sensor. WO2013/142386 (AVNERI) is a similar disclosure focussed on the use of a pressure sensor in the automatic advancement of a guidewire. In particular, WO2013/142386 describes devices and methods for providing endovascular therapy, including facilitating establishment of vascular access, placement of endovascular sheaths, catheter tip localization, and administration of vascular occlusion. The disclosure includes a vessel cannulation device, an expandable sheath, an occlusion catheter, and a localizer each of which may be provided separately or used as part of a system. One example of the disclosure is a vessel cannulation device including: a housing having a distal end with a distal tip and a proximal end; a guidewire lumen passing through the housing and at least the distal tip; a sensor coupled to the guidewire lumen; and an advancing member, which is configured for advancing at least one of a guidewire or a sheath and which is operably coupled to the sensor.

EP0653220 (PHASE MEDICAL INC) relates to an intravascular insertion device in which the needle is spring-loaded for retraction into the device after catheter insertion. Needle retraction is manually triggered by a user.

WO2016/187037 (BARD INC C R) discloses a catheter placement device including an extensible needle safety component.

US5,295,974 (O'LAUGHLIN) discloses a shielded hypodermic needle with an intravascular cannula.

By way of technological background only, it is noted that EP0832663 (BECTON DICKINSON) is directed to a vascular access device for introducing a catheter into a blood vessel, using an introducer needle to penetrate the patient's skin and blood vessel. Once in place, the operator can manually trigger an activating means located within the device to propel past the tip of the needle and into the blood vessel. In particular, EP0832663 describes a vascular access device for introducing a catheter in a blood vessel using a guide advancing mechanism. The device generally comprises a housing, an introducer needle, a catheter, a guide wire and an actuating means. The introducer needle of the vascular access device is used to penetrate the blood vessel. The actuating means is then initiated and the guide wire is steadily propelled into the blood vessel. Once the guide wire is in place, the catheter, which is concentrically placed over the introducer needle and guide wire, is uncoupled from the vascular access device and guided into the blood vessel by the guide wire.

WO2008/005618 (VASCULAR PATHWAYS INC) discloses an intravenous catheter insertion device including a slideable access needle, a guide wire that is manually moved by a user and a release button configured to automatically withdraw one or both of the guide wire and the access needle. In particular, WO2008/005618 describes a catheter insertion device with a housing and an interior space. The device includes an access needle and a guide wire supported by and moveable relative to the access needle. A restraining element is attached to a handle to limit the motion of the guide wire relative to the access needle. Also described is another catheter insertion device with a housing and an interior space. The device includes an access needle and a guide wire channel attached to the access needle. A guide wire is supported by the guide wire channel. A handle is attached to the guide wire to move the guide wire relative to the guide wire support channel. Also described is a method of introducing a catheter into a vessel by inserting a guide wire substantially contained within a housing into a vessel. Next, the method includes advancing a catheter over the guide wire and into the vessel. Next, the method includes withdrawing the guide wire out of the vessel and completely into the housing.

WO 98/19720 A2 describes guide wire placement apparatus for guide wire introduction into blood flow, specifically for main blood vessel catheterization according to Seldinger. The apparatus comprises means for indication of puncture needle readings in blood vessels made as a syringe with barrel made integral with the apparatus housing, means for syringe plunger control, guide wire preinstalled within apparatus outside of barrel and in line with the needle, guide wire control zone permitting guide wire control by the same hand that holds the apparatus and controls plunger. The apparatuses can be made with the option of direct control of guide wire by the operator's finger, or equipped with supplementary control member.

The above devices all require skilled operation to minimise the risk of overpenetration or under-penetration by the introducer needle.

It is an object of the disclosure is to provide a device which reduces or avoids the risk of overpenetration or under-penetration during intravascular catheterisation.

It is a further object of the disclosure to provide semi-automatic insertion of a catheter into a vein once located.

Other objects of the disclosure may include at least one of: reducing the skill required to manipulate a device for intravascular catheterisation, needle tip protection while within the vein, quick retraction of the needle, or the prevention of secondary needlestick once the device is removed.

### Summary of the Disclosure

According to an aspect of the disclosure, there is provided a device for inserting a tube into a body cavity of a patient, the device comprising: a tube; a probe; and a needle configured to enter the body cavity, and to enable entry of the tube and the probe into the body cavity, wherein the device is configured such that the needle, the tube and the probe distally advance in the body cavity after entry of the probe into the body cavity, with the probe advancing distally beyond the needle.

The device of the present disclosure may reduce the likelihood of over- or under-penetration of the needle during insertion of the tube into the body cavity. In particular, the distal advancement of the probe beyond the needle reduces the risk of over-penetration of the cavity by the needle and guides distal advancement of the tube. The distal advancement of the probe after entry into the body cavity also reduces the risk of under-penetration of the cavity by the needle, because the distally advanced probe can serve as a guide for distal advancement of the needle and the tube into the body cavity. The distal advancement of the needle, the tube, and the probe in the body cavity after entry of the probe into the body cavity means that less manipulation is required on the part of a user in order to distally advance the tube into the body cavity. Reducing the amount of user manipulation required reduces the risk of over- or under-penetration owing to the reduced reliance on the user's manual dexterity to distally advance the tube while maintaining the needle in the cavity.

Although, needles are discussed in detail herein, the term "needle" embraces other cutting elements which are suitable for use in tissue penetration.

The distal advancement of the needle, the tube, and the probe in the body cavity after entry of the probe into the body cavity allows the needle (typically formed of a material such as stainless steel) to act as a rigid support for insertion of the probe and tube into the body cavity, thereby ensuring, for example, that the tube is inserted in a correct direction in the body cavity. In addition, the distal advancement of all three components (needle, tube, probe) allows a simple mechanism to be used to drive the distal advancement of the components into the body cavity. In particular, as each of these components is distally advanced, a drive mechanism can be used to link the components and drive the movement of each of the components. The use of a drive mechanism that links the components (needle, tube, probe) and drives their movement allows the components to be distally advanced using a smooth movement.

The device of the present disclosure significantly increases the rate of first-time tube insertion and may decrease the risk of body cavity or body tissue injury and may reduce the skill level required for operation. With the tube being inserted correctly more often than with conventional device designs, this may lead to less delay in implementing treatment.

Devices in accordance with the disclosure may comprise a drive mechanism providing semi-automatic control means and which is arranged so that forces within or on the mechanism balance to maintain the needle, tube, and probe in a stable initial pre-use or storage condition, and, in use, the forces vary to cause the probe to advance relative to the tube on or after entry of the probe into the cavity; and to advance the probe relative to the needle. The term "semi-automatic" means in relation to the control means that the control means, and specifically the drive mechanism, functions substantially without user intervention, to control the positions of the probe and needle relative to the tube (e.g., catheter) during insertion of the tube. The term "semi-automatic" may also relate to the automatic retraction of the needle and/or probe after insertion of the tube. Devices including such a mechanism is advantageous in that it enhances the smooth operation of the device which may reduce, for example, the risk of under-penetration compared to known devices.

The device of the disclosure may result in significant savings in terms of number of devices required per successful catheterisation, less delay in implementing treatment, fewer complications, and/or a better experience for both patients and clinicians. The device of the disclosure may be economically advantageous for healthcare providers by lowering costs of cannula usage, in terms of number of devices required per successful catheterisation. The device of the disclosure may reduce rates of complications. The device of the disclosure may enhance patient and clinician satisfaction levels. The device of the disclosure may less require less training for users. The device of the disclosure may reduce any risk of secondary needlestick. The device of the disclosure may provide more immediate or reliable insertion of the probe extending into the vein. The device of the disclosure may provide more immediate or reliable feedback to a user, the feedback coming from the probe extending in a body cavity such as a vein.

By not requiring a mechanical trigger mechanism as used in certain devices described in the patent literature, the device of the disclosure may be smoother to operate in inserting tubes into body cavities leading to, for example, more reliable intravascular catheterisation. According to another aspect of the disclosure, there is provided an applicator device according to claim 67 for inserting a tube into a body cavity of a patient. This is essentially a device according to the disclosure without a tube. The applicator device may be supplied or used with a conventional tube such as a catheter to insert the tube as disclosed herein in relation to a device in accordance with the disclosure.

According to a further aspect of the disclosure there is provided a method of inserting a tube into a body cavity of a patient according to claim 68. The patient may have at least one condition inhibiting correct insertion of a tube such as a catheter, the condition(s) being at least one of obesity, comparatively small blood vessels, fragile blood vessels, deep blood vessels, collapsed blood vessels, tortuous blood vessels, skin colour, chronic disease, venous depletion, or lymphoedema. Advantageously the device or applicator device may be operated with one hand of a user. The method of the disclosure may be performed by a robotic machine. For example, in the method a device according to the disclosure, or an applicator device, and an associated tube, is held by a robotic machine.

According to further aspects of the disclosure, there are provided an intravascular cannula, comprising:
a) a body to be held by a user;
b) an elongate introducer needle having a tip;
c) an elongate catheter (i.e., one example of a tube) in association with the introducer needle for introduction into the lumen of a blood vessel (i.e., one example of a body cavity of a patient), the catheter having a connection end and a tip;
d) an elongate probe in association with the introducer needle;
e) semi-automatic control means (i.e., an example of a drive mechanism) arranged to: maintain the needle, catheter, and probe in a stable pre-use condition; and cause the probe to advance relative to the catheter on or after contact of the probe with the lumen of the blood vessel; and also, methods for the use of an intravascular cannula as described above. For example, the intravascular cannula can be used in a method of intravascular catheterisation of a patient having a blood vessel, the method comprising contacting the intravascular cannula or catheter applicator and associated catheter as the case may be with the skin of a patient, permitting the needle to enter the skin (or tissue) under the control of the semi-automatic control means, permitting the probe to advance relative to the catheter after contact of the probe with the lumen of the blood vessel, retracting the needle, retracting the probe, and leaving the fully inserted catheter in the lumen of the blood vessel.

### Brief Description of the Drawings

Devices and methods of operation of devices in accordance with the disclosure will now be described, by way of example only, with reference to the following drawings, Figures 1 to 71 in which:
Figure 1 is an elevation of a device in accordance with the disclosure in an initial condition or stage of operation shown in relation to tissue including a blood vessel;
Figure 1A is a perspective view of the device of Figure 1 in accordance with the disclosure in use in an initial tissue-engaging condition or stage of operation;
Figure 1B is a horizontal longitudinal cross-section through the device of Figure 1 illustrating a locking arrangement between a slider and a casing of the device in an unlocked condition;
Figure 2 is an exploded view showing a portion of the device's mechanism, including a probe spool for controlling the relative motion of the cannula needle and probe relative to a catheter in more detail;
Figure 2A is a perspective view of the assembled mechanism shown in Figure 2;
Figure 2B is a perspective view of the device of Figure 1 in longitudinal cross section (in the initial tissue-engaging condition);
Figure 2C is a perspective detail view, including a partial cutaway, of a portion of the device of Figure 1;
Figure 3 is a longitudinal vertical cross-section through the device of Figure 1 in the initial tissue-engaging condition;
Figure 3A is a detail view of a portion of Figure 3;
Figure 3B is a schematic free body diagram illustrating the forces acting on the probe spool in the initial tissue-engaging condition shown in Figure 1;
Figure 3C is a further schematic illustrating the effect of the forces involved in the initial tissue-engaging condition shown in Figure 1;
Figure 4A shows the device of Figure 1 ready for use;
Figure 4B is a detail view of the tip region of the device in the condition shown in Figure 4A;
Figure 5A shows the device in the tissue-engaging condition further stage of operation;
Figure 5B is a detail view of the tip region of the device of Figure 1 in the condition shown in Figure 5A;
Figure 6A shows the device in a further stage of operation as the needle, probe and catheter enter a vein;
Figure 6B is a detail view of the tip region of the device of Figure 1 in the condition shown in Figure 6A;
Figure 7A shows the device of Figure 1in a further stage of operation;
Figure 7B is a detail view of the tip region of the device in the condition shown in Figure 7A;
Figure 8A shows the device of Figure 1 in a further stage of operation;
Figure 8B is a detail view of the tip region of the device in the condition shown in Figure 8A;
Figure 9A shows the device of Figure 1 in a further stage of operation;
Figure 9B is a detail view of the tip region of the device in the condition shown in Figure 9A;
Fig 9C is a horizontal longitudinal cross-section similar to Figure 1B of the device illustrating the locking arrangement between slider and casing in a locked condition in the stage of operation shown in Figure 9A;
Figure 10A shows the device of Figure 1 in a yet further stage of operation;
Figure 10B is a detail view of the tip region of the device in the condition shown in Figure 10A;
Figure 11A shows a device of Figure 1 in a further stage of operation;
Figure 11B is a detail view of the tip region of the device in the condition shown in Figure 11;
Figure 12A shows the device of Figure 1 in a further stage of operation;
Figure 12B is a detail view of the tip region of the device in the condition shown in Figure 12A;
Figure 13A shows the device ofFigure 1 in a final stage of operation;
Figure 14 is a perspective detail view of another cannula in accordance with the disclosure showing an alternative probe arrangement;
Figure 15 is a perspective detail view of another cannula in accordance with the disclosure showing another probe arrangement;
Figure 16 is a perspective view of certain components of a device in accordance with a second embodiment of the invention showing the needle carrier and control spool of the device;
Figure 16A is a detail view showing in particular the control spool of the device of Figure 16;
Figure 17 is a perspective view showing further components of the device of Figure 16;
Figure 18 is another perspective view showing further components of the device of Figure 16;
Figure 19 is another perspective view illustrating components of the semi-automatic control mechanism of the device of Figure 16;
Figure 20 is a perspective view illustrating the complete device of Figure 16;
Figure 21 is a longitudinal cross-section of the device of Figure 16 in an initial condition of use in relation to skin, tissue and a vein of a patient;
Figure 21A is a schematic view illustrating the forces acting on various components of the semi-automatic control mechanism of the device of Figure 16 in the condition shown in Figure 21;
Figure 22 is a longitudinal cross-section of the device of Figure 16 in a subsequent condition of use in relation to skin, tissue and a vein of a patient;
Figure 22A is a schematic view illustrating the forces acting on various components of the semi-automatic control mechanism of the device of Figure 16 in the condition shown in Figure 22;
Figure 23 is a longitudinal cross-section of the device of Figure 16 in a subsequent condition of use in relation to skin, tissue and a vein of a patient;
Figure 23A is a schematic view illustrating the forces acting on various components of the semi-automatic control mechanism of the device of Figure 16 in the condition shown in Figure 23;
Figure 24 is a longitudinal cross-section of the device of Figure 16 in a subsequent condition of use with the probe extending along the lumen of the vein of the patient;
Figure 24A is a schematic view illustrating the forces acting on various components of the semi-automatic control mechanism of the device of Figure 16 in the condition shown in Figure 24;
Figure 25 is a longitudinal cross-section of the device of Figure 16 in a subsequent condition of use with initiation of retraction of the probe and needle relative to the catheter;
Figure 25A is a schematic view illustrating the forces acting on various components of the semi-automatic control mechanism of the device of Figure 16 in the condition shown in Figure 25;
Figure 26 is a perspective view of components of a device in accordance with a third embodiment of the invention primarily showing a rail element of the device, the device being shown incomplete in Figure 26 and complete in Figure 32;
Figure 27 is a perspective view showing further isolated components of the device of Figure 26 including a needle carrier and needle;
Figure 28 is a perspective view showing further components of the device of Figure 26 including a slider;
Figure 29 is another perspective view showing further components of the device of Figure 26 including a catheter;
Figure 30 is a perspective view showing further components of the device of Figure 26 including cam plates of the semi-automatic control mechanism of the device;
Figure 31 is a perspective view showing further components of the device of Figure 26 including a handle/casing of the device;
Figure 31A is a detail view of the device of Figure 26 showing the position of a clip when the device is in an extended condition in a later stage of operation;
Figure 31B is a schematic transverse cross-sectional view showing the relationship between the needle, catheter, and a groove formed by the rail in an initial condition of device of Figure 26;
Figure 32 is a perspective view of the device of Figure 26 in an initial condition or stage of use in relation to skin, tissue, and a vein of a patient (which are shown in cross-section);
Figure 33 is a longitudinal cross-section of the device of Figure 26 in the initial condition of use;
Figure 33A is a schematic illustrating forces in the mechanism in the device in the condition of Figure 33;
Figure 34 is a longitudinal cross-section of the device of Figure 26 in a subsequent condition in use;
Figure 34A is a schematic illustrating forces in the mechanism in the device in the condition of Figure 34;
Figure 35 is a longitudinal cross-section of the device of Figure 26 in a subsequent condition in use showing extension of the probe in the lumen of the vein;
Figure 35A is a schematic view illustrating the forces acting on various components of the semi-automatic control mechanism of the device of Figure 26 in the condition shown in Figure 35;
Figure 36 is a longitudinal cross-section of the device of Figure 26 in a subsequent condition in use showing extension of the needle and catheter in the lumen of the vein;
Figure 37 is a longitudinal cross-section of the device of Figure 26 in a subsequent condition in use showing the full extension of the catheter in the lumen of the vein;
Figure 37A is a detail view of the device of Figure 26 showing the position of the clip in the condition of device as shown in Figure 37;
Figure 37B is a schematic transverse cross-sectional view showing the relationship between the needle, catheter, and groove in the condition of the device of Figure 37;
Figure 38 is a longitudinal cross-section of the device of Figure 26 in a subsequent condition in use;
Figure 38A is a detail view of the device of Figure 26 showing the position of the clip in the condition of the device shown in Figure 38;
Figure 38B is a schematic view showing the relationship between the needle, catheter, and groove in the condition, or stage of operation, of the device shown in Figure 38:
Figure 39 is a perspective view of major components of a partially assembled device in accordance with a fourth embodiment of the invention;
Figure 39A is a perspective view (with partial cutaway) showing some of the components shown in Figure 39 in more detail;
Figure 39B is a perspective view showing some of the components shown in Figure 39 in more detail;
Figure 39C is a perspective view (with partial cutaway) showing some of the components shown in Figure 39 in more detail;
Figure 40 is a perspective view of the partially assembled device of Figure 39 showing further components of the device;
Figure 41 is a perspective view of the partially assembled device of Figure 40 showing further components of the device;
Figure 42 is a perspective view of the assembled device of Figure 40 in an assembled condition ready for use;
Figure 42A is a detail transverse cross-section of a portion of the assembled device;
Figure 43 is a longitudinal cross-sectional view of the assembled device of Figure 39 to 42A in an initial condition of use and in relation to a patient (shown in cross-section);
Figure 44 is a further longitudinal cross-sectional view of the device of Figure 43 in a later stage of use;
Figure 44A is a free body diagram showing forces involved in the stage of use of Figure 44;
Figure 45 to 49 are a series of further longitudinal cross-sectional views of the assembled device of Figure 43 in later sequential stages of use;
Figure 50 to 52 are a series of part cross-sectional detail views illustrating the operation of the slider and clip of the assembled device of Figure 43 in use;
Figure 53 is a perspective view with a partial cutaway showing partially assembled components of a device in accordance with a fifth embodiment of the disclosure;
Figure 53A is a perspective view of components (principally the probe and a control wheel) of the device of the fifth embodiment of the disclosure;
Figure 53B is a perspective view of components of the device of the fifth embodiment of the disclosure;
Figure 54 is a perspective view of the device of the fifth embodiment of the disclosure in a partially assembled state;
Figure 55 is a perspective view of the device in accordance with the fifth embodiment in a ready for use condition;
Figure 55A is a lateral cross-section through a portion of the device of Figure 55 showing details of the device;
Figures 56 to 63 are a series of longitudinal cross-sectional views of a device of the fifth embodiment of the disclosure in a sequence of steps of inserting a catheter into a vein of a patient;
Figure 64 is perspective view of a partially assembled device in accordance with a sixth embodiment of the disclosure;
Figure 64A is another perspective view of the partially assembled device of Figure 64 showing further components;
Figure 64B is another perspective view of the partially assembled device of Figure 64 showing further components;
Figure 64C is another perspective view of the partially assembled device of Figure 64 showing further components;
Figure 64D is another perspective view of the partially assembled device of Figure 64 showing further components;
Figure 64E is another perspective view of a fully assembled device of Figure 64 showing further components; and
Figure 65 to 71 are a series of longitudinal cross-sectional views of the device in accordance with the sixth embodiment of the disclosure in a sequence of steps of inserting a catheter into a vein of a patient.

### Description

In the description below of devices in accordance with various embodiments of the invention some features specifically described in relation to certain embodiments, such as the colouring of components, may be applicable to other embodiments even if not specifically described in relation to those other embodiments.

Implementations of the present disclosure are explained below with particular reference to intravascular catheterisation, in which a tube in the form of a catheter is inserted into a body cavity in the form of a blood vessel. It will be appreciated, however, that the devices described herein are also capable of insertion of other forms of tube into other forms of body cavity, such that the devices described herein have general applicability to the insertion of a tube into a body cavity. For example, the devices described herein may also be used for intercostal drains, pericardiocentesis, suprapubic catheter insertion, intrathoracic surgical port site insertion, and intraabdominal surgical port site insertion. Other examples will be apparent to the skilled person. Moreover, although the implementations of the present disclosure are explained below with reference to operation by a user, it will be appreciated that the devices described herein may also be operated by a robot.

### A device (part of an invention to which the present European patent / application relates)

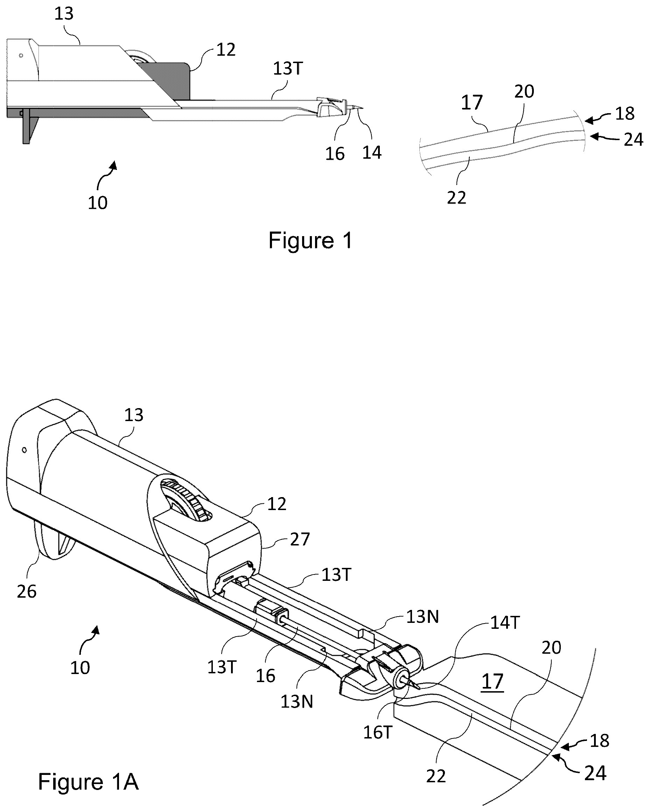

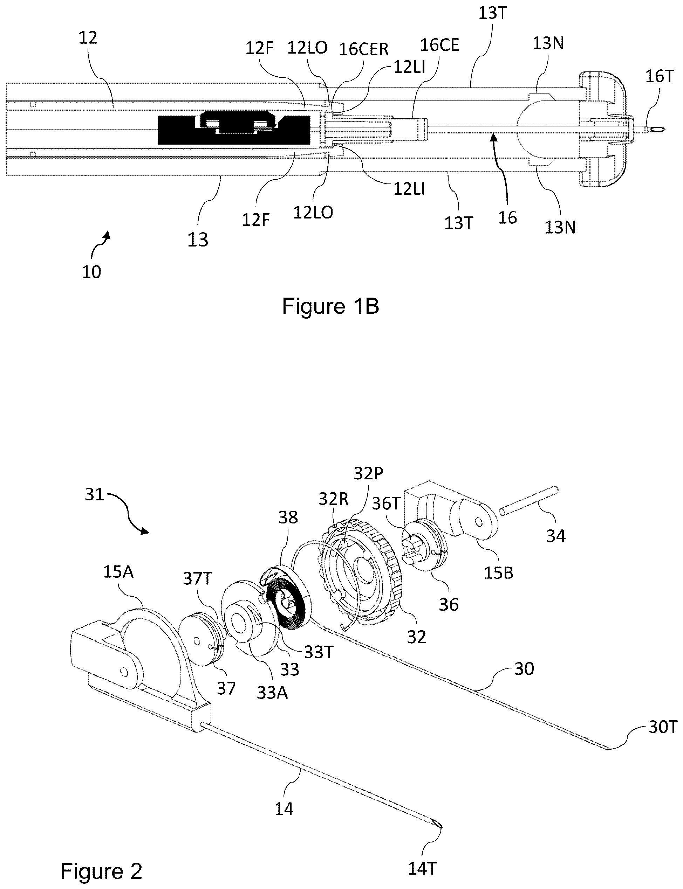

A device 10 in accordance with a first embodiment of the disclosure is shown, by way of example only, in Figures 1 to 15. In accordance with normal practice, the device 10 is supplied sterile for single use. As shown in Figure 1, the complete device 10 principally comprises: a manually grippable slider unit 12, formed from a plastics material, a casing 13, which is also formed from plastics material, and is slidably engaged with the slider unit 12, hollow metal introducer needle 14, and a tube in the form of a polymer tubular catheter 16, disposed concentrically about the needle 14. The device 10 is generally constructed and arranged so that the introducer needle 14 and catheter 16 can be advanced, through the skin 17 of tissue 18 of a patient, through the proximal wall 20 of a body cavity such as a blood vessel, here vein 24, and into the lumen 22 of that blood vessel 24, so that the catheter 16 is left correctly inserted in the blood vessel 24.

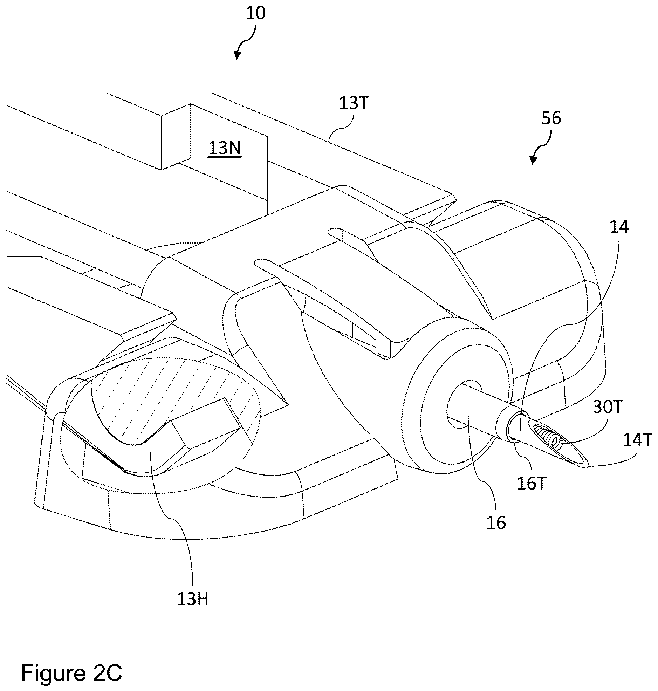

A flexible metal probe 30 (obscured in Figure 1) is arranged within the needle 14 in the configuration shown in Figure 1. As shown in Figure 2C, the probe 30 is formed from a tightly coiled metal wire (formed, for example, in the manner of a Seldinger guide wire). Other forms of probe which are longitudinally relatively stiff, so as to transfer the tissue force Fₜᵢₛₛᵤₑ (referred to below), but which can move within a blood vessel such as a vein without overpenetration when deployed within that blood vessel are contemplated. It will be appreciated that the probe 30 could be made from a range of materials using a variety of manufacturing techniques to create a member that is longitudinally stiff while it is in the initial/tissue-engaging condition and thus able to transfer the force from the tissue to the mechanism of the device. It is contemplated that the probe may become flexible as it advances so that it can follow a nonlinear path along the vein without risk of puncturing the wall of the vein. This reduces the risk of overpenetration of the needle and guides the advance of the catheter. The required probe characteristics are achieved by virtue of the tightly coiled wire that is held within the needle. This allows the tissue force (Ftissue) to be relayed back up the probe to the mechanism. Without the relatively stiff needle, it will be appreciated by the skilled addressee, that the probe may buckle so there may be an interdependence between these components to achieve the desired result. For example, the blunt-ended probe may be formed from stainless or nitinol wire. The wound coil may have a diameter of 0.34 - 0.45 m.m. according to the lumen diameter of the needle which depend on the gauge of the catheter as discussed below. Diameters of about 0.3 m.m. are preferred. In more detail, Figure 1A shows that the slider 12 is formed to define a rear grip 26 and a forward grip 27 which enhance a user's grip on the slider 12. The casing 13 extends forwardly to form a track 13T. Opposed notches 13N are formed by the inner walls of the track 13T towards the forward end of the track for engaging locking clip elements formed by the slider 12 as described below in relation to Figure 1B.

The polymer catheter 16 is of generally conventional construction and comprises (as shown in Fig1B) an elongate thin tube ending in tip 16T and a connection end 16CE which allows connection to an infusion line after catheterisation. The connection end 16CE forms a rim 16CER. The connection end 16CE of the catheter 16 may or may not include a port or valve for the injection of drugs in a conventional manner. Other catheter designs may be used such as integrated or closed systems e.g., the Becton Dickinson Nexiva^{™} closed catheter system.

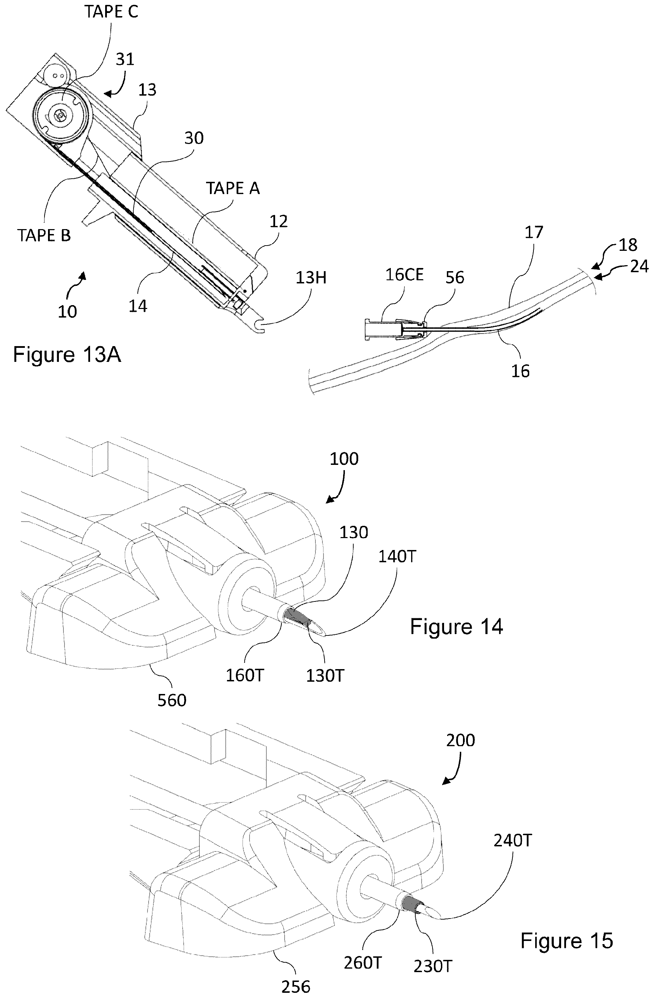

In this embodiment, the probe 30 is arranged concentrically within a hollow needle 14 and the needle is in turn arranged concentrically within the catheter 16 The skilled addressee will appreciate that other permutations are possible with such concentrically arranged elements. In one embodiment the needle and probe may not be concentrically arranged but instead may lie alongside each other (see Figure 14). Alternatively, the needle in this and other devices in accordance with the disclosure may be arranged concentrically within the probe and the catheter is then concentrically arranged around the probe (see Figure 15), within or around the catheter. In some of these arrangements, and indeed in other devices in accordance with the disclosure, the needle might not have a circular cross-section or be hollow. For example, it may be solid and provided with cutting surfaces as, for example, in a surgical needle. The probe may be blunt at its distal end whereas the needle (or cutting element) is relatively sharp. It can be understood that the needle (or cutting element) will part the skin/tissue/etc, while the blunt end of the probe will transmit the resistive force of the tissue to the mechanism until the point of breakthrough into the lumen of the blood vessel.

Fig 1B also shows in more detail the locking arrangement between the slider 12 and casing 13 in an unlocked condition and the initial retention of the catheter 16 by the slider. The slider 12 is formed to include a pair of elongate resilient clip fingers 12F which in turn each terminate in inward-facing and outward-facing lock lugs 12LI and 12LO respectively. The notches 13N defined by the opposed inner walls of casing track 13T are arranged to receive the corresponding outward facing lugs 12LO in a locked condition for the slider 12 and casing 13 as discussed below. In the initial condition shown in Figure 1 and 1B, and described below, one or more coupling elements in the form of the inward-facing lugs 12LI engage and hold the catheter rim 16CER and therefore restrain the catheter 16.

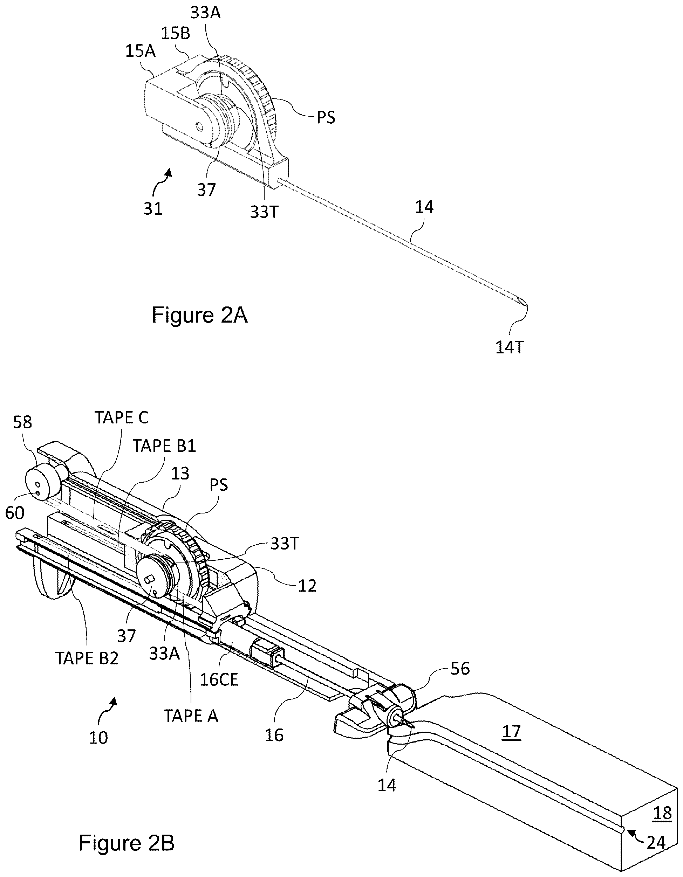

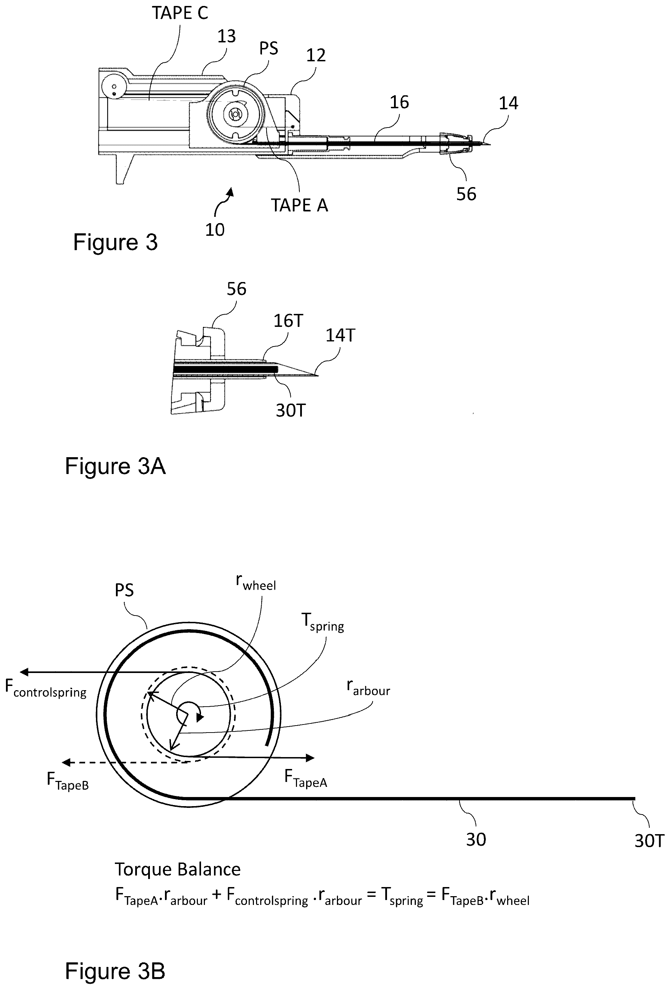

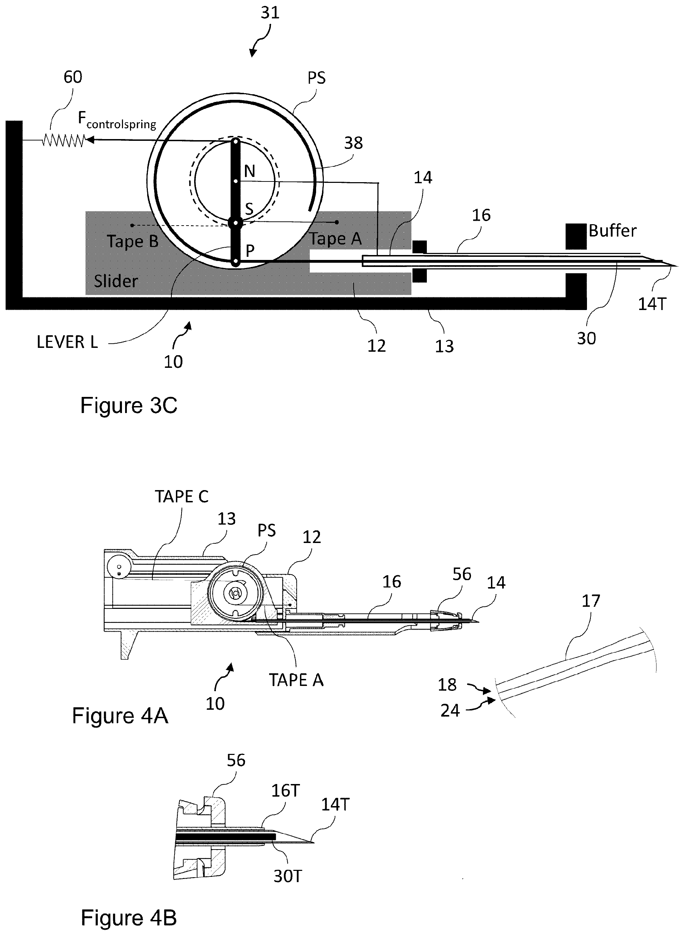

A part of the control mechanism 31 (also referred to herein as a drive mechanism) for controlling the movements of the needle 14 and the probe 30 relative to the catheter 16, is shown in Figure 2, 2A and 2B. The needle 14 is held by a needle carrier 15, which comprises two needle carrier parts 15A and 15B (as shown in Figure 2A). The needle carrier 15 is movable longitudinally in relation to the slider unit 12 to move the needle 14 towards or away from a patient. The mechanism 31 includes a rotatable element in the form of a knurled probe spool element 32 of radius of about 10 m.m., and probe spool cap 33, together forming a probe spool PS, which is mounted for rotation about the same axis as wheels 36, 37 on axle 34 which are in turn of a smaller radius (r_{wheel}) - about 5m.m. - than the probe spool PS and mounted for rotation about the axle 34. As discussed by Harty, E. *supra* catheters are provided commercially in a variety of different gauges. Accordingly, it will be appreciated by the skilled addressee that in other devices in accordance with the invention, the gauge of the catheter may be varied in accordance with normal practice, and the diameters of the probe and needle adjusted accordingly. The probe spool cap 33 fits within the knurled probe spool element 32 and is held fast for rotation therewith by respective cooperating lugs and recesses. The probe spool cap 33 has a round arbour 33A which is of a slightly smaller diameter than the wheels 36, 37. The arbour 33A bears a tooth 33T which projects from the surface of the arbour. The probe spool PS is free to rotate between the two needle carrier parts 15A and 15B. The probe spool PS encloses a watch-type main spring 38, one end of which engages a protrusion 32P formed by the probe spool 32 and the other end of which engages tangs 36T, 37T formed at the centres of the wheel 36, 37. The main spring 38 will therefore tend to apply a torque T_{spring} between the wheels and the probe spool PS. The torque Tspring is generally consistent. The proximal end of probe 30 also fits inside a rim 32R within the circular knurled probe spool element 32 with a radius of about 10 m.m. and is fixed to that element, thereby mechanically linking the proximal end of the probe 30 to the rotatable element. The proximal end of the probe 30 which is retained inside the rim 32R naturally tends to unwind. The mechanism 31, which forms part of the semi-automatic control means is mounted for longitudinal translational movement on the slider 12, which is, in turn, arranged to move in longitudinal translation on track 13T which extends from the casing 13. The probe spool PS may be coloured differently/patterned to the casing 13 or slider 12 so that its rotation may be visually highlighted to a user. This may provide a visual indication of the operation of the device to the user. The mechanical linkages (e.g., tapes or strings) which connect the mechanism 31 for controlling the movement of the needle 14 and probe 30 relative to the catheter 16 are shown in particular in Figure 2B and Figure 3. The strings or tapes are made of a limited or non-stretch material. In the embodiment shown, tapes are used which are made, for example, from PTFE (Teflon). Tape A extends rearwardly to connect a forward portion of the slider unit 12 to the arbour 33A of the probe spool cap 33. Specifically, Tape A has a series of longitudinal slots LS (obscured) one of which engages the tooth 33T on the arbour 33A of the probe spool cap. Tape B1 extends forwardly to connect rearward portion of the slider 12 to the wheel 36 (the wheel 36 is obscured in Figure 2B). Corresponding Tape B2 similarly extends forwardly to connect the slider 13 to the other wheel 37. The main spring 38 is arranged so that the torque between the probe spool PS and the wheels 36,37 (torque Tspring) result in the Tapes A, and B1 and B2 being in tension. A further Tape C extends rearwardly from about the arbour 33A to the casing via the rotatable wheel 58 to which it is attached. Tape C also has a series of longitudinal slots LS which are spaced apart and dimensioned also to engage the tooth 33T on the arbour 33A. The wheel 58 is mounted for rotation on the casing 13 and is biased by an internal control torsion spring 60 (obscured) to hold the attached Tape C in tension. The forward end of slider 12 engages the rearward end 16CE of the catheter 16, the catheter 16 being received within a recess defined by the forward end of the slider.

As shown in Figure 2C, the respective forward ends of the catheter 16 and the introducer needle 14 are received within and supported by a buffer 56 which is detachably fixed on the forward end of casing track 13T. The principal function of the buffer is to engage against or abut a body surface such as the patient's skin/tissue 17/18 to hold the device 10 stationary during insertion of the needle 14, probe 30 and catheter 16. In this way, the buffer 56 provides a datum (also referred to herein as a support leg) for the operation of the device 10. More specifically, the buffer 56 is hooked on hooks 13H (one obscured in Figure 2C) formed at the end of casing track 13T and is kept in a hooked condition on the track whilst the needle 14 extends through the buffer 56.

In the stable pre-use condition as illustrated in e.g., Figure 3, the torques acting on the probe spool PS are balanced. The equation shown in Figure 3B shows that the relatively large forces in Tapes A and B produced as a result of the watch main spring 38 are countered by a relatively small force in Tape C. This is a result of the small difference in radius between the wheels 36,37 and the arbour 33A. In the stable pre-use condition, the tensional force applied by control torsion spring 60 through the Tape C (F_{controlspring}) to the probe spool cap arbour 33A balances the relatively large torque produced by the substantially constant torque watch main spring 38 (T_{spring}) and the assembly is balanced in equilibrium. In this state, the probe tip 30T, needle tip 14T and catheter tip 16T generally align as shown in Figure 3A. In the stable pre-use condition, the slider unit 12 cannot move rearwards relative to the casing 13 due to a backstop at the rear end of track 13T. This allows for a small amount of tension in Tape C to create the balance described above.

By way of further illustration, Figure 3C shows the probe spool PS again in the initial pre-use condition represented schematically by a lever L (overlaid in black). It can be seen that this schematic lever L pivots around point S, the effective pivot created by the linkages of Tapes A and B which are connected to the slider 12. The effective linkage of the probe is shown at P and the effective linkage of the needle 14 is shown at N. Thus, it can be seen that if the needle 14 is pushed rearward in the direction of arrow D, the probe 30 will be advanced and *vice versa.* The control spring 60 will contract or extend accordingly and return the mechanism 31 to the initial pre-use condition.

The normal operation of the device 10 in introducing a catheter 16 into the blood vessel, specifically into the lumen of a vein 24 of a mammalian subject 102 through skin 17 and underlying tissue 18 is shown in Figures 4 to 13 and described in order below.

In the initial pre-use condition of the cannula shown in Figure 4A and B, the slider 12, casing 13, and mechanism 31 are in the respective positions shown, and the probe 30 is within the needle 14 and catheter 16 and aligned as shown in particular in Figure 4B. The connection end 16CE of the catheter 16 is held by the inward-facing lugs 12LI as described above.

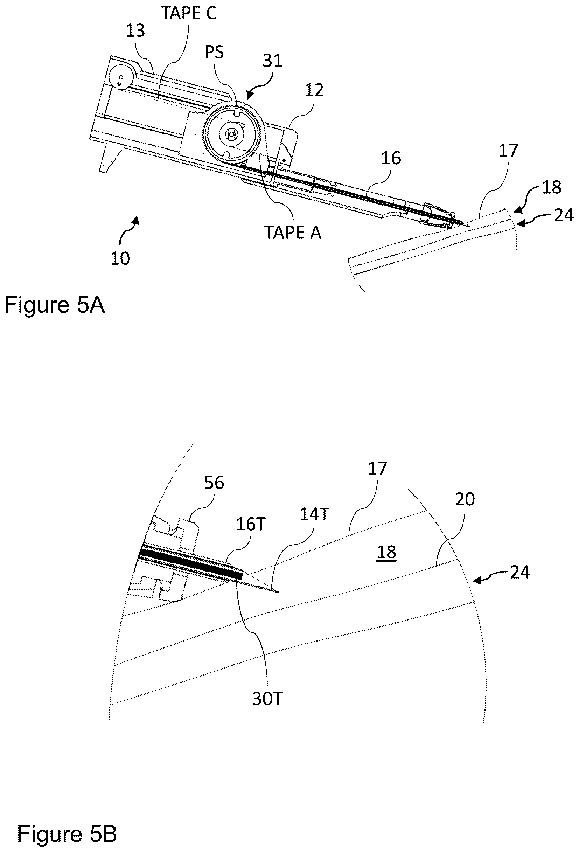

In the tissue-engaging condition shown in Figure 5A and B, the tip 14T of the introducer needle 14 is inserted into the tissue 18, and the buffer 56 abuts the surface of the tissue 18 - specifically the skin 17 of the patient - holding the device 10 (more specifically the casing 13) stationary against the patient. At this point, the forward end 16T of the catheter 16 also abuts the skin 17. As the slider 12 advances relative to the casing 13/buffer 56, the needle 14 cuts through the skin 17 and tissue 18 and Tape C unwinds from the rotatable wheel 58 and the control torsion spring 60 applies a biasing force to the probe spool PS by increasing the force (F_{controlspring}) applied to the probe spool PS. This urges the probe spool PS to rotate in a first direction, that is, contraclockwise (in the sense of Figure 3B and Figure 3C), but this rotation is inhibited by the contact between the probe tip 30T and the skin 17 (and therefore the tissue 18) which results in force Fₜᵢₛₛᵤₑ.

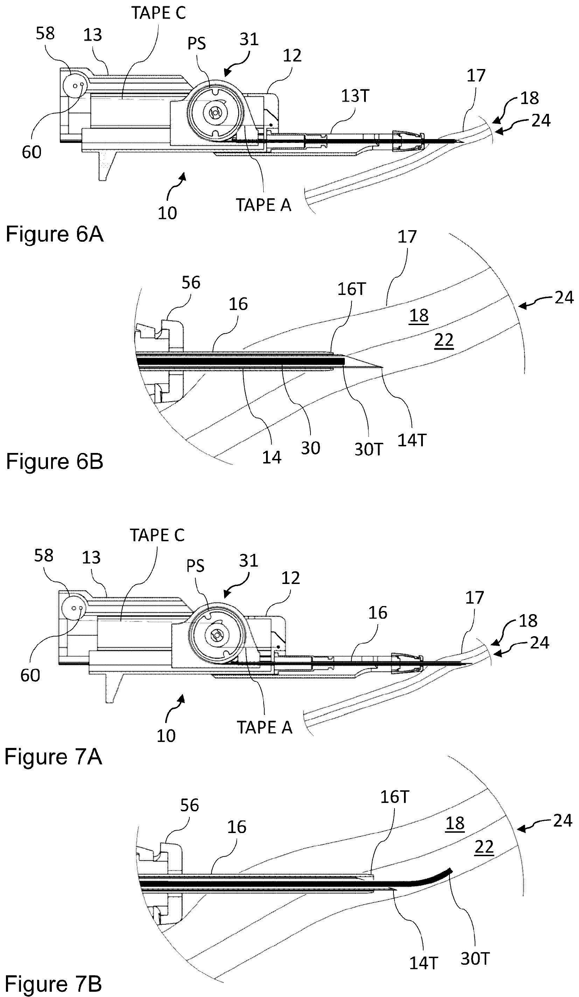

Once the needle tip 14T breaks into the lumen 22 of the vein 24, as shown in Figure 6A and B, the force from the tissue Fₜᵢₛₛᵤₑ reduces, and the balance of the arrangement is disturbed. The probe spool PS is rotated contraclockwise by the biasing force (i.e., tension in Tape C caused by the control spring 60), and this causes the probe 30 to be advanced distally relative to the catheter 16 and also the needle tip 14T to retract relative to the catheter 16 as shown in Figure 7A and B (in other words, the catheter 16 advances distally relative to the distal advancement of the needle tip 14T).

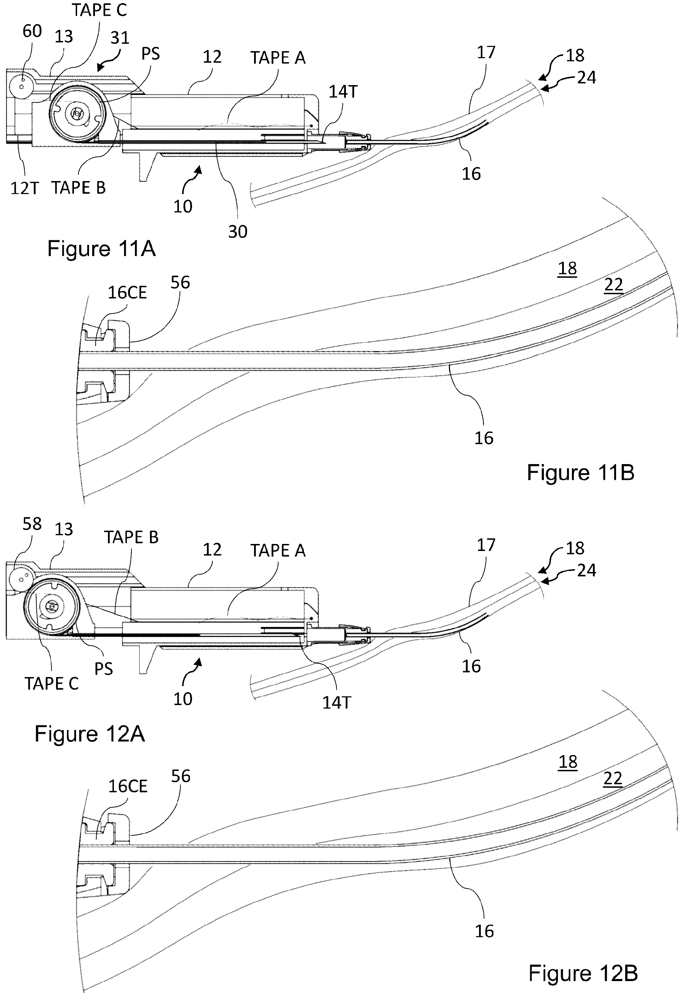

Thereafter, the relative radii of the wheels 36,37 and the probe spool PS (i.e., the probe spool 32/probe spool cap 33 combination) providing the mechanical linkages between the probe spool PS and the probe 30, needle 14 and catheter 16, control the relative rate of advance of the probe 30, needle 14 and catheter 16. In particular, the probe 30 extends along the lumen 22 of the vein 24 faster than the catheter 16 as shown in Figure 8A and B. This extension of the probe 30 significantly reduces the risk of, or prevents, the needle 14 from penetrating or extending through the opposite (distal) wall 25 of the vein 24. Additionally, it may prevent under-penetration i.e., insertion of the needle/catheter into tissue outside the vein. Furthermore, as also shown in Figure 8B, the needle tip 14T retracts relative to the catheter 16 and is quickly covered by the advancing catheter. Once the catheter 16 is fully inserted, its connection end 16CE engages the buffer 56 clipping the components firmly together by means of cooperating detents. The probe spool PS rotates sufficiently in a contraclockwise (i.e., first) direction (in the sense of Figure 3B) for the end of Tape A to be released from the tooth 33T of arbour 33A (as shown in Fig 9A). With the detachment of Tape A from the arbour 33A, the balance of the arrangement is now lost, and the wheels 36,37 rotate contraclockwise (in the sense of Figure 3B) and probe spool PS rotates in a second direction, that is, clockwise (in the sense of Figure 3B), drawing mechanism 31 including the needle carrier 15 backwards (to the left in the orientation shown in Figure 3) and quickly withdrawing the needle 14 inside the forward portion of the casing 12 (as shown in Figure 12).

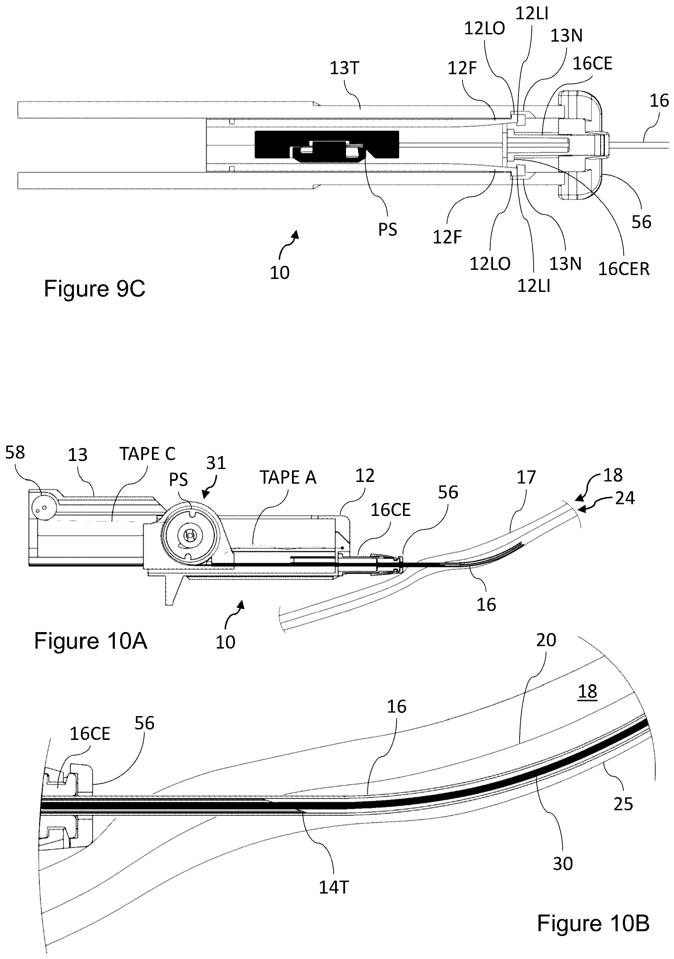

Figure 10A illustrates how tapes C and B1/B2 take over when tape A is released from the arbour 33A, sending the mechanism 31 rearward. In Figure 10A, the wheels 36,37 are running rearwards on track surfaces 12T within the slider. In Figure 11A, the wheels 36,37 have transferred onto the track of the casing. Furthermore, even though the wheels 36,37 have passed over the point where Tapes B1 & B2 are connected to the slider 12, the needle carrier 15 continues to be pulled backwards because the force in Tape C is greater than in Tapes B1 & B2. This is due to the smaller radius of the arbour 33A compared to that of the wheels 36,37 represented by: ${T}_{spring} = {F}_{TapeC} . {r}_{arbour} = {F}_{TapesB 1 & B 2} . {r}_{wheel}$

As shown in Figure 9C, slider 12 and casing 13 have moved relatively such that the outward facing slider lugs 12LO now engage the notches 13N of the casing track 13T. The slider 12 is thus held by this inter-engagement in a locked condition relative to the casing 13 and cannot be returned. The outward engagement of the outward-facing lugs 12LO with the notches 13N of the casing track has the effect of the inward-facing lugs 12LI releasing the connection end 16CE of the catheter 16.

As shown in more detail in Figure 9B, the connection end 16CE of the catheter 16 engages with the buffer 56 to lock the two components together.

In the condition shown in Figure 12A, the needle carrier 15 has moved to its rearmost position. In this locked condition, the needle 14 is safely retracted within the confines of the casing 13. The probe 30 is also retracted within the needle 14. The wheel 58, which connects with Tape C to the probe spool PS is provided with an end limit so that the wheel does not keep rotating under the force applied by the main spring 38.

As shown in Figure 13A, with the retraction of the needle the buffer 56 can then be simply removed from the casing 13 by unhooking it from hooks 13H which was not possible when the needle 14 was extending. With the catheter 16 being correctly inserted (i.e., the so inserted catheter is "fully deployed") the other components of the cannula 10 can then be safely disposed of in conventional manner. As noted above, the buffer 56 is now fixed to the connection end 16CE of the catheter 16, which is received within the buffer, and the buffer now essentially forms a conventional catheter "wing" which facilitates taping of the catheter 16 (specifically the connection end 16CE) to the skin 17 of the patient to hold it in place in conventional manner. The buffer 56 may be designed to reduce slippage in relation to the skin. For example, the buffer 56 may be produced in a tacky plastics material (such as highly plasticised PVC) or it may be physically formed with projections (such as hooks) which increase skin engagement. In other embodiments, the buffer 56 is not separable from the casing 13 i.e., it is formed integrally with the casing and the whole casing is removed and disposed of after insertion of the catheter.

In the mechanism described above, the probe spool, wheels, and tape arrangements effectively create a "lever" that, along with the springs, controls the motion of the probe and needle relative to the catheter. This desired controlled motion can be created in a wide range of different ways using different combinations of known springs, dampers, lever and gear mechanisms. For example, the probe spool, wheels, and tape "lever" described could be replaced by a simple physical lever, or the tapes and wheels replaced with a rack and pinion or other known mechanical devices that could be used to create the desired motion control. Such alternative control mechanisms are described in the later embodiments.

Figure 14 shows a portion only of another cannula 100 according to the disclosure. Catheter 100, which is generally the same as cannula 10 described above, including corresponding needle tip 140T and catheter tip 160T passing through buffer 560 save that the end of the probe 130 has a semi-blunt end 130T. The end of the probe 130T may be formed by a tip on a probe or by a shaped end to a probe, and that the needle is crescent shaped at least towards the needle tip 140T, and that the needle and probe lie side by side. The relative bluntness of the probe and relative sharpness of the needle can be altered to control the relative forces occurring during passage through the tissue.

Other configurations are possible. For example, Figure 15 shows a portion only of another device 200 according to the disclosure. Device 200, which is generally the same as device 10 described above, including corresponding needle tip 240T and catheter tip 260T passing through buffer 256 save that probe 230 is arranged outside needle 240.

### Another device (part of an invention to which the present European patent / application relates)

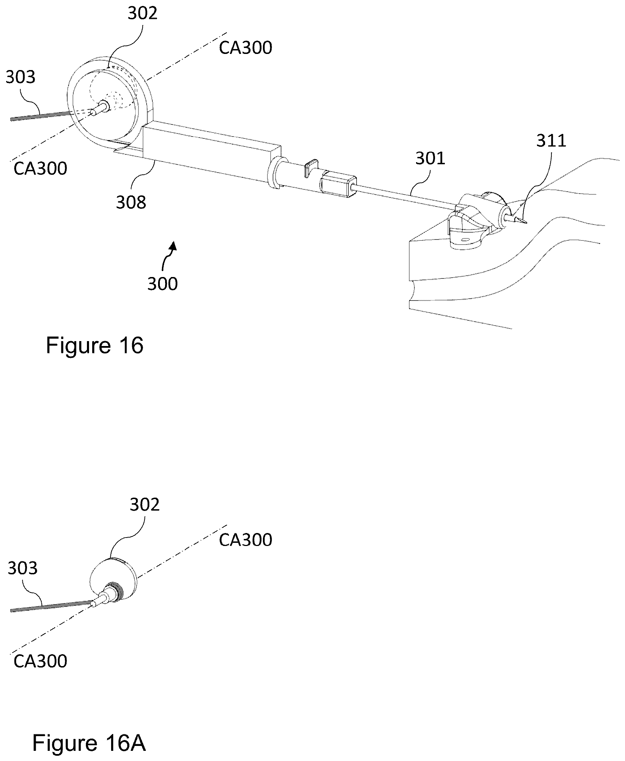

Another device 300 for inserting a tube in the form of a catheter 301 which is in accordance with second embodiment of the disclosure is shown in Figures 16 to 25. In the device 300 according to this embodiment, the tapes of the first embodiment are replaced with mechanical linkages in the form of rack and pinion gears, and the torque springs and rollers are replaced with a mechanical linkage in the form of a single elastic string. Devices in accordance with this embodiment may be easier to manufacture. It will be appreciated from the following description that the mechanism in this embodiment also uses the lever principle established in the first embodiment. Furthermore, the mechanism of this embodiment also embodies the principle of balancing forces established in the first embodiment. The device 300 includes a drive mechanism comprising a rotatable element in the form of a control spool 302 which is formed as shown (e.g., in dotted outline in Figure 16, and in Figure 16A) to provide a spiral groove around its perimeter with varying radius from the centre axis CA300 of the control spool. The elastic string 303 which is made of, for example, natural or synthetic rubber is connected to the end of the groove and wound around the control spool 302 such that it is sitting in the groove of the control spool. The control spool 302 is mounted on the needle carrier 308 (comparable to the needle carrier 15 of the first embodiment) holding the needle 311. The control spool 302 is free to rotate about its central axis CA300.

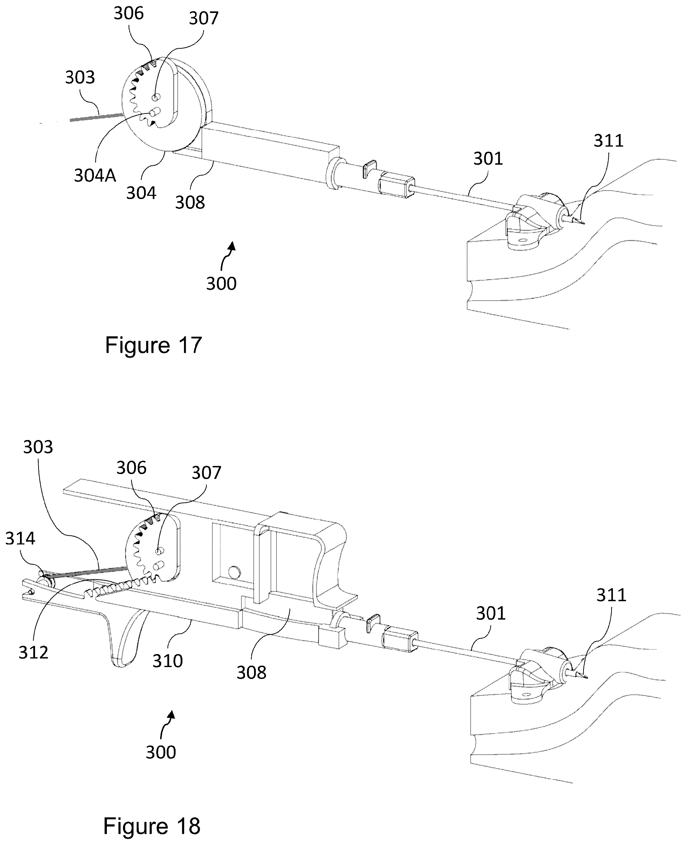

As shown in Figure 17, a probe spool 304 is connected to the control spool 302 so that they rotate as one on an axle 304A. The probe spool 304 is comparable to probe spool element 32 of a previous embodiment and has an internal rim (comparable to feature 32R in the first embodiment) to which the proximal end of a probe 305 is attached. The rim and probe 305 are not visible in this view but the probe/probe spool arrangement is comparable to that of the first embodiment. The radius of probe spool 304 is about 10mm in this embodiment. The probe spool 304 includes an integral toothed spur gear 306 element and a pin 307 extends from a face of the probe spool 304. In this embodiment (but not necessarily, as other profiles are contemplated) the radius of the spur gear 306 follows the shape of a logarithmic spiral starting with a small radius compared to the probe spool radius and ending with a radius comparable to or larger than the radius defined by the probe as it sits in the internal rim of the probe spool 304. In this embodiment, the internal rim of the probe spool 304 has a radius of about 10mm, and this is the radius defined by the probe at that point. It will be appreciated that the logarithmic spiral starts much smaller than 10mm and ends about equal or greater than 10mm.

As shown in Figure 18, needle carrier 308 sits within a slider 310 (comparable to previous slider 12) which engages the catheter 301 in a similar way to the first embodiment. The logarithmic spur gear 306 on the probe spool 304 engages with a straight inclined gear rack 312 which is integrally formed as part of the slider, forming a rack and pinion arrangement. The elastic string 303 passes around a roller 314 rotatably mounted at the rear of the slider 310 and is then connected to a forward part of the slider 310 such that there is tension in the elastic string 303.

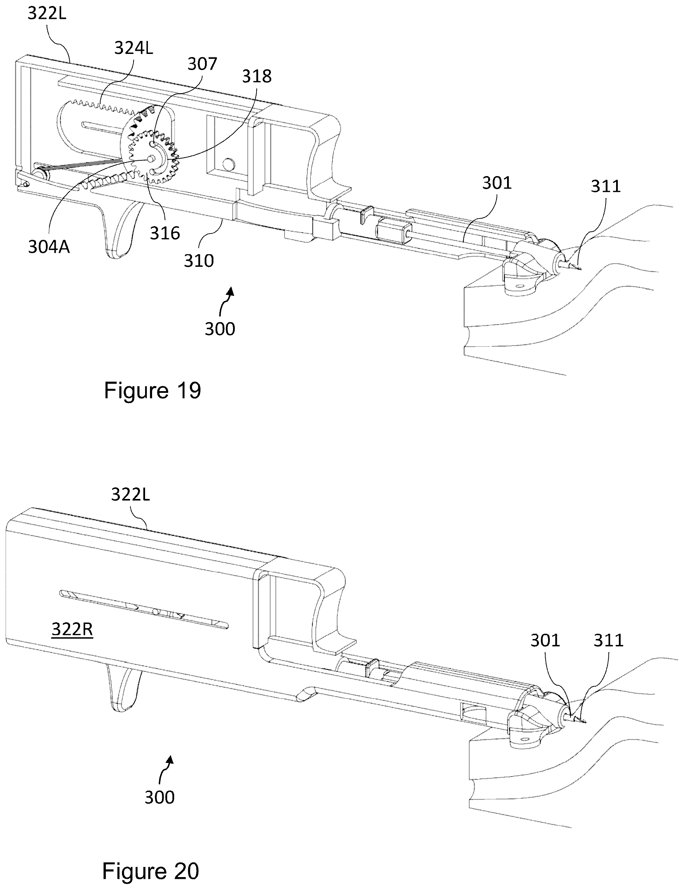

As shown in Figure 19, a first spur gear 316 is mounted on the axle of the probe spool 304. This first spur gear 316 is free to rotate around the probe spool axle within the limits defined by a slot 318 in the spur gear 316 which acts against the pin 307 extending from the adjacent face of probe spool 304. A corresponding second spur gear 319 is mounted on the far side of the assembly, on the same axle protruding out of the control spool 302 and is similarly limited in its rotation by a similar pin formed on the control spool 302 moving within a slot 320 (not shown) which is defined by the further spur gear 319. The slots 318, 320 are mirrored about the central plane of the device 300 (not seen in this view). The slider 310 is free to slide within a two-part outer casing 322 (comparable to the casing 13 of the first embodiment). The left-hand side of the casing 322L is shown in Figure 19. The second spur gear 319 engages in a rack 324L formed within the outer casing 322 and the axle travels in a groove 326 defined within the adjacent wall of the casing 322. Similarly, the first spur gear 316 engages in a mirrored rack 324R (not shown in this view) on the right-hand casing 322R.

The complete assembled device 300, including the right-hand casing 322R, is shown in Figure 20.

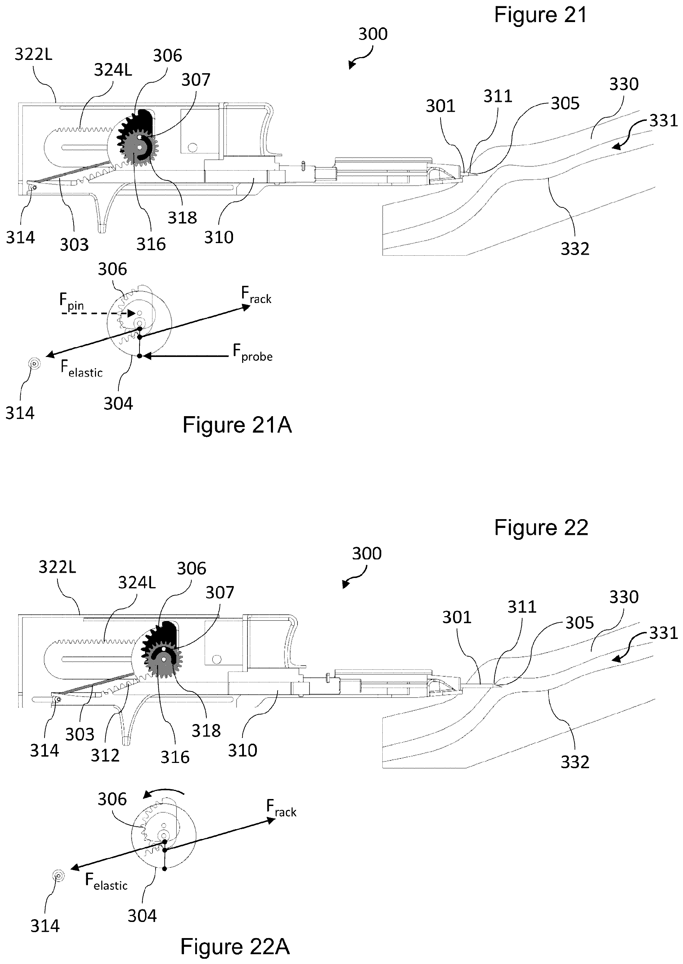

The operation of the device 300 to insert catheter 301 through tissue 330 into the lumen 331 of vein 332 in a series of sequential steps is shown in the accompanying Figures 21 to 25A. The initial stage of operation (comparable to Figures 4A and 5A of the first embodiment) is shown in Figure 21. As the needle 311, probe 305, and catheter 301 are inserted into the tissue 330, the force on the probe 305 (F_{probe}) is balanced by the tension force in the elastic string 303 (F_{elastic}) with the probe spool 304 effectively pivoting about the rack and pinion gear arrangement of this embodiment. This balance in the control mechanism will be maintained as the needle 311, probe 305, and catheter 301 advance through the tissue 330. An advantage of this arrangement is that, as the roller 314 is rotating on the slider 310, the tension in the elastic 303 remains constant during the tissue cutting or advancement stage - i.e., the force does not increase as the slider 310 advances.

Prior to the needle 311 entering the tissue 330, and as illustrated by the schematic view of Figure 21A, the probe spool 304 is kept in balance by the force exerted by the pin 307 against the end of slot 318 in the spur gear 316. In this condition, the slider 310 is in a backstop position relative to the casing 322 and the spur gear 316 cannot rotate as it is engaged in the rack 324R. It will be appreciated that as the slider 310 is advanced into the tissue 330, the spur gear 316 will rotate anticlockwise (in the orientation of Fig 21) creating a gap between the slot 318 and the pin 307. This allows the balancing force to transfer from the pin 307 onto the probe 305.

The "breakthrough" stage of the needle 311, probe 305, and catheter 301 into the lumen 331 of the vein 332 (comparable to the step of Figure 6A in the first embodiment) can be seen in Figure 22. As the needle 311, probe 305 and catheter 301 break through into the lumen 331 of the vein 332, the force on the probe 305 (F_{probe}) reduces and no longer balances the tension force in the elastic string 303(F_{elastic}) as reflected in Figure 22A.

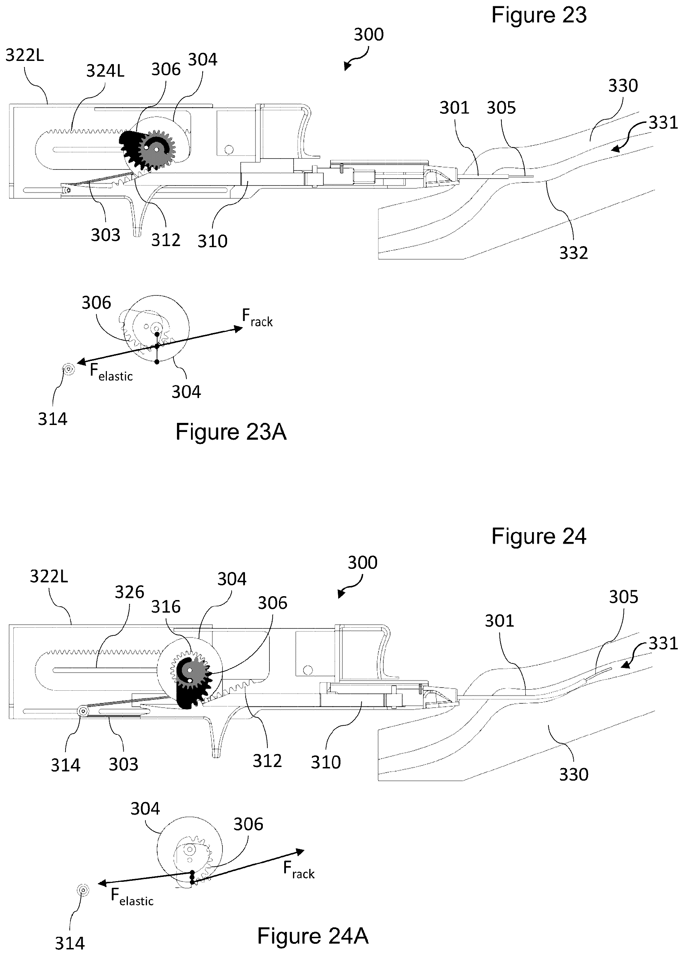

The probe spool 304 will now pivot about the rack and pinion gear arrangement in an anticlockwise direction (in this view) and will rotate and travel along the rack 312 of the slider 310 as shown in Figure 23. As the probe spool 304 rotates the probe 305 advances relative to the catheter 301. The radius of the logarithmic spur gear 306 is relatively small compared with the radius of the probe spool 304 at this point of connection with the rack 312. This means that the probe 305 advances quickly with reference to the catheter 301. The needle 311 moves backwards relative to the catheter 301 generally as described above in relation to the first embodiment. As the probe spool 304 continues to rotate, the elastic string 303 engages along the groove of the control spool 302 which is, as noted above, is directly connected to the probe spool 304. The radius of this groove from the common axis CA300 increases until the effective radius of the elastic in the groove is the same as the radius of the logarithmic gear 306 on the probe spool 304. At this point, and as reflected in Figure 23A, the forces are balanced so the probe spool 304 stops rotating. The spur gear 316 will, however, continue to rotate as the slider 310 continues to advance the catheter 301 into the lumen 331 of the vein 332.

The next stage of operation is shown in Figure 24 and is also reflected in the schematic of Figure 24A. As the slider 310 advances, the axle will eventually hit the end of the groove 326 in the casing 322L. At this point, the probe spool 304 will continue to rotate driven by the engagement of the slider rack 312 with the logarithmic gear 306. During this stage, the radius of the logarithmic gear 306 is large compared to the diameter of the probe spool so the rate of advance of the probe 305 is less than at the breakthrough. The advantage of this arrangement is that the probe 305 does not advance much further than the end of the catheter 301 (compare to previous Figure 9A of the first embodiment). Alternatively, the shape of the logarithmic gear 306 could be arranged so the catheter "catches up" with the probe 305 so that they are aligned at the end position. Once the slider 310 reaches an end position, the logarithmic gear disengages from the rack 312 causing the needle and needle carrier to quickly retract in a comparable way to the tape A releasing from the arbour 33A in a previous embodiment. Note the roller 314 transfers so that it is now rotating at the end of grooves defined in either side of the casing. This increases the tension in the elastic string 303 as the slider 310 advances. This increased tension is required for the needle 311 to retract.

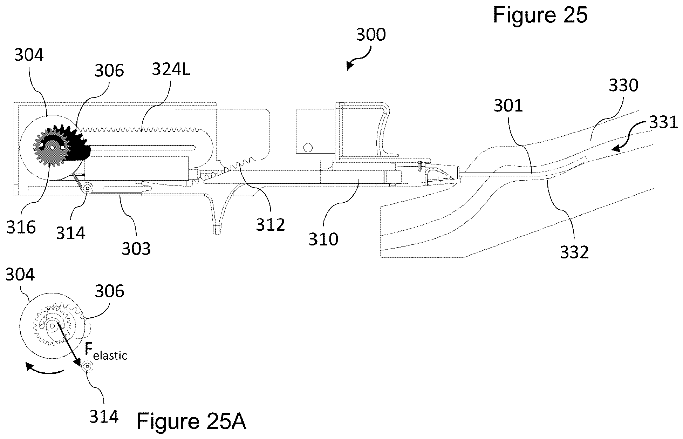

Turning now to the condition shown in Figure 25 and referring also to the schematic view of Figure 25A, once the logarithmic gear 306 disengages from the rack 312, the probe spool 304 is unbalanced and rotates clockwise driven by the elastic string 303 around the control spool 302. The pins stop against the slots of the spur gears which in turn drives the needle carrier 308 rearwards along the rack 324 in the casing. This results in the needle 311 and probe 305 retracting from the catheter 301. The device 300 is then removed from the catheter 301 generally as described before in relation to the first embodiment. The clips holding the catheter to the slider act generally in the same way as before. It will be noted that in this embodiment the string 303 is arranged so that the force balanced by the probe spool 304 is roughly constant throughout the tissue cutting stage by virtue of the roller 314 first moving with the slider 310 then being transferred to the casing to increase tension for the subsequent needle retraction phase. It will also be noted that in this embodiment, the use of a logarithmic gear with a varying effective radius enables the probe 305 to move quickly during breakthrough in the vein and then slow down relative to the catheter 301. This results in a probe that does not significantly overshoot the catheter in the end position.

### A further device (part of an invention to which the present European patent / application relates)

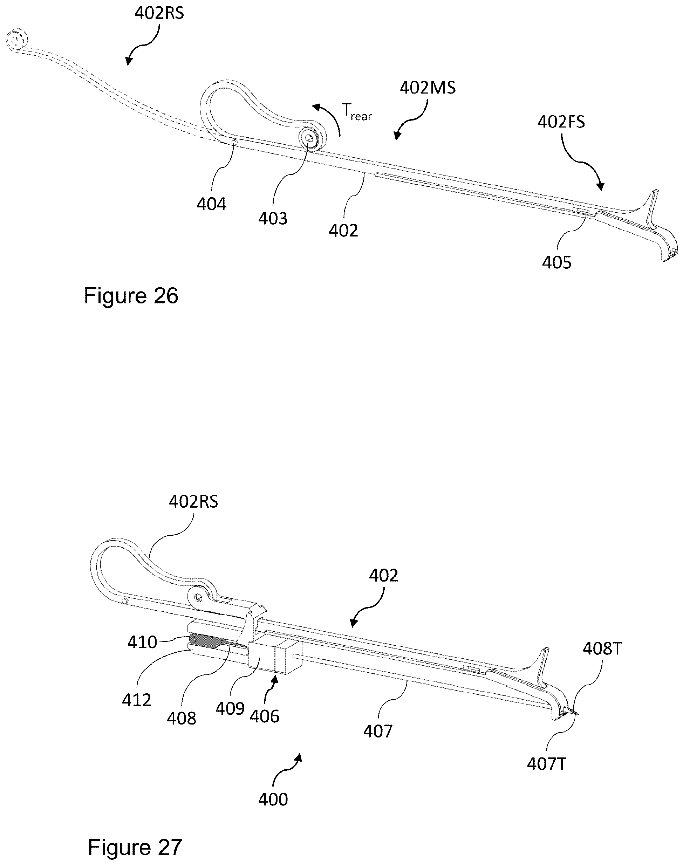

A further device 400, its components, and its method of operation to insert a tube in the form of a catheter into a blood vessel of a patient are shown in Figures 26 to 38B. In this embodiment, which is still based on the principles of the virtual lever and balancing of forces which were established in the first embodiment, the function of the tapes of the first embodiment, or that of the rack and pinion gears of the second embodiment described above, is replaced with mechanical linkages in the form of flexible plastic springs that provide both the balancing forces and the virtual lever of the semi-automatic control mechanism. Creating the virtual lever from flexible resilient plastic springs gives a particularly smooth action compared to using gear or pin linked components. The automated needle retract function of the previous embodiments is removed and the needle is manually retracted. The probe spool is replaced with a drive mechanism comprising a rotatable element in the form of a cam mechanism acting against a probe tooth moving longitudinally. These features simplify the mechanism of the semi-automatic control means and associated production cost of the device of this embodiment. This embodiment may therefore be easier and cheaper to manufacture and assemble.

The device 400 includes an elongate rail component 402, shown in Figure 26, which is made from a plastics material, and which comprises a front section 402FS with a forward-facing tip 402T, that in operation engages with the skin of the patient providing a datum (or support leg) for the device, an intermediate middle rail section 402MS on which various movable components of the device slide, and a rear spring section 402RS, which supports a boss 403. The rear spring section 402RS is formed nominally straight (shown by a dashed outline). The shape, thickness, and material properties of the rear spring section 402RS are such that when it is bent into position (as shown by the solid outline), the spring section imparts a torque (Tᵣₑₐᵣ) on the central boss (anticlockwise as shown). The rail component 402 is shown in Figure 26 as a single injection moulded plastic (e.g., nylon) component but could also be made, for example, from spring steel or spring steel over-moulded with plastic. A stop pin 404 and a notch 405 are included in the structure of the elongate rail component 402 as shown in Figure 26.

A needle carrier 406, which carries a hollow introducer needle 407 is shown in Figure 27. The needle carrier 406 (which is functionally similar to the needle carrier 15 described above in relation to the first embodiment) is arranged to slide loosely on the rail 402. The needle carrier 406 also includes a transparent chamber 409 at the proximal end of the needle 407 which is in flow communication with the needle and constitutes a "flash chamber" as it fills with blood once the needle 407 enters a patient's vein. The device 400 further includes a wire probe 408 which is made of a flexible coiled wire similar to the probe 30 in the first embodiment. The probe 408 has a plastic tooth-shaped element 410 over-moulded onto it at its proximal end. The tooth-shaped element 410 slides longitudinally on a rear rail 412 extending rearwards from the needle carrier 406. As shown in the initial condition of the device 400 generally represented in Figure 27, the probe 408 is arranged so its distal tip 408T is just aligned with the sharp end 407T of the needle 407.

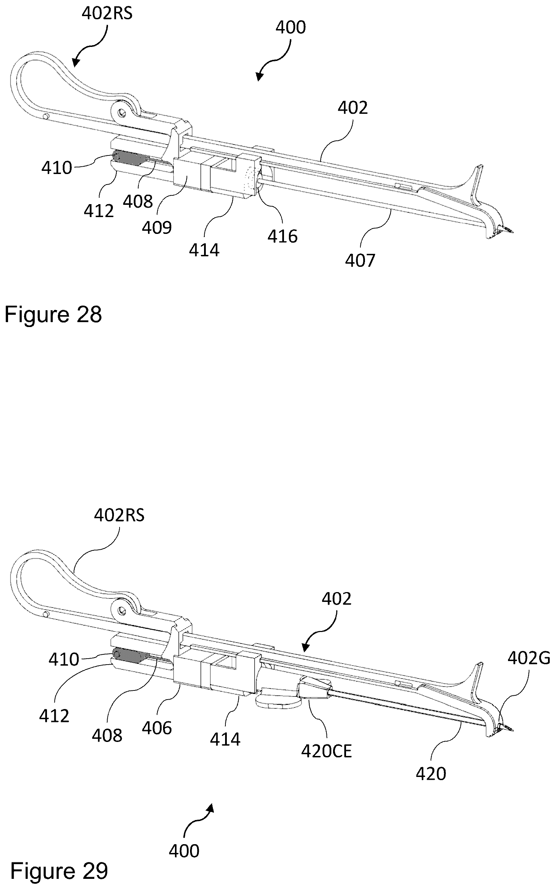

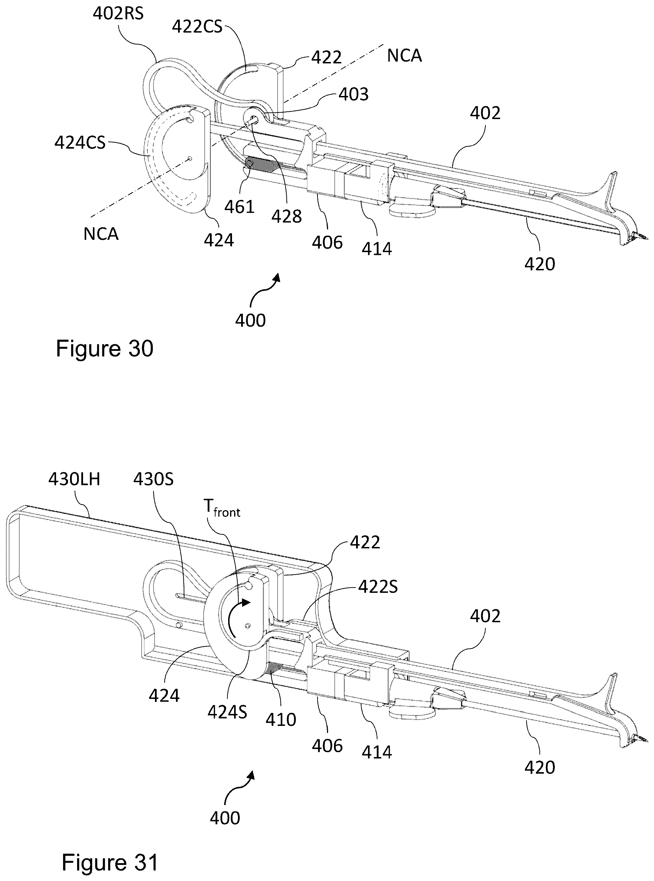

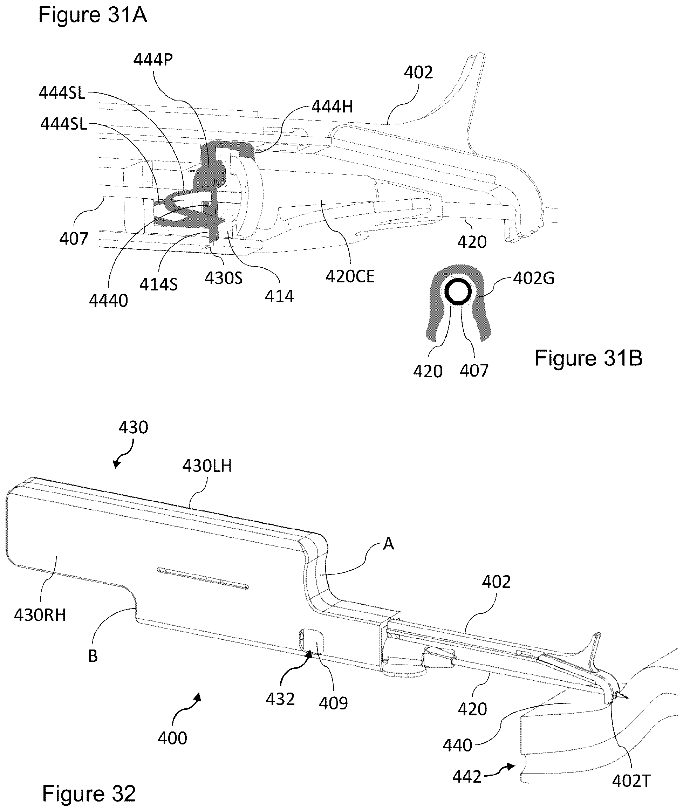

A slider 414, which is mounted to slide longitudinally along the rail 402, is shown in Figure 28 arranged forward of flash chamber 409. The needle 407 is arranged to pass through an aperture defined by a central boss 416 within the slider 414. The slider 414 houses a coupling element in the form of a pressed spring steel clip 444 (obscured in Figure 28 but shown in detail in Figure 31A).

As shown in Figure 29, a catheter 420 is mounted over the central boss 416 within the slider 414 and clipped in place using the spring steel clip 444 described later. The catheter is essentially conventional in construction. The distal end of the catheter 420 rests within a groove 402G at the distal end of the front section of rail 402 (shown in detail in Figure 31B). The proximal end of the catheter 420 has a moulded plastic connection end 420CE

As can be seen in Figure 30, a left-hand cam plate 422 is connected to the boss 403 of the rear spring 402RS such that the cam plate 422 and the boss 403 rotate as one around the axis of the needle carrier NCA shown in Fig 30. A corresponding right-hand cam plate 424 is also attached to the boss 403. This right-hand cam plate 424 mirrors the left-hand cam plate 422 and rotates with it about axis NCA. The cam plates 422, 424 are connected to the boss 403 and secured using an axle pin 428. The axle pin 428 protrudes on each side from the respective outer surfaces of the cam plates 422, 424 and supports the resulting semi-automatic control mechanism of the device within a slot 430S (not shown in Figure 30) on the handle/casing. The camming surfaces 424CS of the cam plate 422, and 422CS of cam plate 424 act against pins 461 protruding from the tooth shaped element 410 fixed to the probe 408.

A user grippable handle/casing 430, which is shown in Figure 31, is arranged to slide longitudinally along the rail 402. The left-hand side 430LH of the casing 430 is shown here. The handle 430 encases the slider 402 and the two components are connected with the clip 444 (not shown in Figure 31). One end of the axle pin 428 which connects the cam plates 422, 424 and rear spring boss 403 locates in a slot 430S formed by the handle 430. This arrangement is mirrored for the other end of the axle pin 428. The cam plates 422,424, boss 403 and needle carrier 406 can slide longitudinally within this slot. Each cam plate 422, 424 has an associated front spring 422S, 424S respectively. The proximal end of each front spring 422S, 424S is connected to the associated cam plate 422, 424. Each front spring 422S, 424S is wrapped around a projecting surface of its associated cam plate 422, 424 and the distal ends of the front springs 422S, 424S are fixed to adjacent inner surfaces of handle 430. The front springs 422S, 424S are formed such that they provide a clockwise (as shown in Figure 31) torque (T_{front}) to their respective cam plates 422, 424 as indicated.

The steel spring clip 444, referred to above, is shown in full shading in Figure 31A as being formed with an upright plate 444P which defines a hole 444O through which the needle 407 passes. The clip 444 connects the catheter 420 to the slider 414 by means of a plate 444P which extends upwards and bends over to form a flat upper surface ending in downwardly extending hook 444H. The lower portion of plate 444P extends downwards through a slot 414S formed in the slider 41 and engages in a corresponding slot 430S handle 430. This connects the slider to the handle. The clip 444 has a pair of spring legs 444SL on either side of the plate 444P that exert a downwards force on the slider urging the plate 444P with the hole 444O and hook 444H upwards.

The righthand side 430RH of the handle/casing 430 is shown in Figure 32 in position, connected to the left-hand side 430LH in the assembled device 400. In use, the handle 430 is gripped by a right-handed user with forefinger at A and thumb at B. In this embodiment, a window 432 is optionally included in the casing 430 to give a user a view through to the flash chamber 409 within the needle holder 406 so that blood flowing through the needle 407 after successful needle entry into the lumen 442 of the vein can be detected. This view of blood in the flash chamber may give additional comfort to a user but is not necessary for correct operation of the device. The front section of the rail 402T is shaped so it sits against the skin 440 of the patient and provides a datum for the semi-automatic control mechanism of the device 400 during the insertion of the catheter into the lumen of a vein 442.

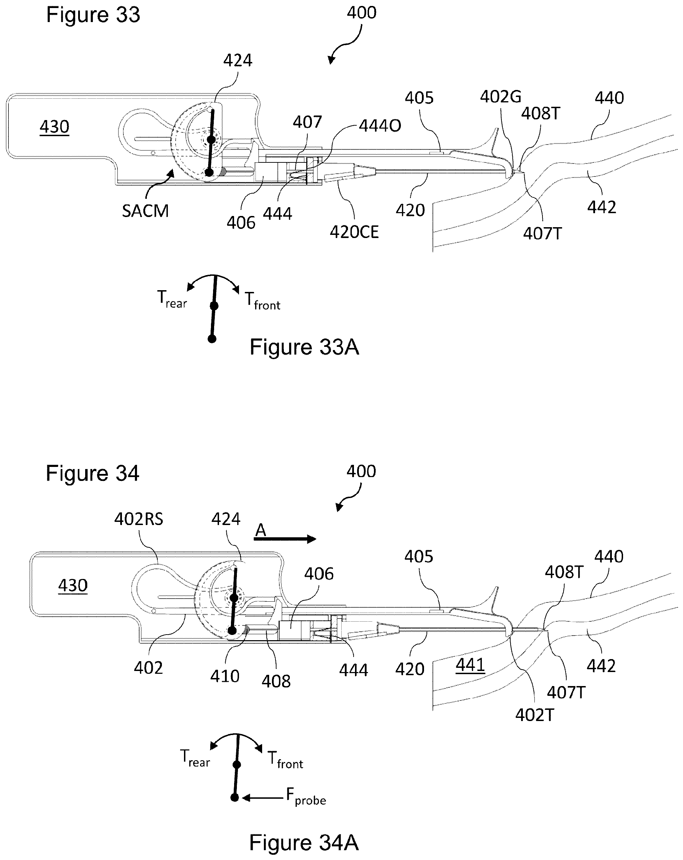

The operation of the device 400 in the insertion of the catheter 420 into vein 442 is now described with reference to the further drawings Figure 33 to 38B which illustrate successive conditions of the device in operation. In the "initial" condition, or stage of operation, shown in Figure 33, the tip 402T of the front section of the rail 402 sits against the skin 440 of a human patient. As noted above, this contact provides a datum to the semi-automatic control mechanism of the device 400. The clip 444 which connects the catheter 420 to the slider 424 and the slider to the handle/casing 430 is seen in clip "Position A". In Position A, (while the slider 414 moves from its initial position up to the notch 405), the upper surface of the clip 444 is running along the lower surface of the rail 402, which holds the clip down against the spring legs 444SL. The hole 444O in the plate 444P is therefore clear of the needle 407 allowing the needle to slide completely smoothly without touching the sides of the hole 444O. The upper hook 444H of the clip prevents the catheter connection end 420CE from moving forwards off the slider boss 416. The two elements are therefore connected. The plate 444P extends downwards through a slot414S in the slider to engage a corresponding slot 430S in handle/casing 430, connecting the two elements. Finally in Position A, the catheter 420 is supported by the forward section 402FS of the rail as shown in Figure 31B. The catheter 420 passes through a groove 402G in the forward section of the rail 402 such that it can slide smoothly longitudinally. The flexible catheter tube 420 is restrained in the groove 402G because the rigid needle 407 within the lumen of the catheter in this condition prevents the catheter tube from squeezing out.

As represented schematically in Figure 33A, the cam plates 422, 424 are held in equilibrium in the stage shown in Figure 33 as torque (Tᵣₑₐᵣ) imparted by the rear spring section 402R balances the torque (Tfront) imparted by the front springs 422S and 424S. The pins 461 protruding from tooth 410 of the probe 408 sits against the camming surfaces 422CS, 424CS with the distal end 408T of the probe 408 in line with the distal end 407T of the needle 407. The semi-automatic control mechanism (which is generally indicated as SACM) is advanced along the rail 402 by pushing the handle 430 forwards in the direction of arrow A of Figure 34. The tip 402T of the forward section of the rail continues to sit against the skin 440 of the patient providing a datum to the mechanism. The catheter 420, needle 407, and probe 408 then advance through the skin 440 and underlying tissue 441. The needle carrier 406 moves forwards relative to the rail 402. This changes the shape of the rear spring section 402RS of the rail, (as represented in Figure 34) increasing Tᵣₑₐᵣ. Tᵣₑₐᵣ is no longer equal to T_{front} which tends to urge the cam plates 422,424 in an anticlockwise (in this view) direction. However, the force of the blunt proximal end of the probe 408 acting against the skin 440 and underlying tissue 441 (T_{force}) is transmitted along the length of the probe and transmitted via the probe tooth 410 to the cam plates 422, 424, as reflected in Figure 34A. The cam plates 422, 424 are therefore in equilibrium and do not rotate.

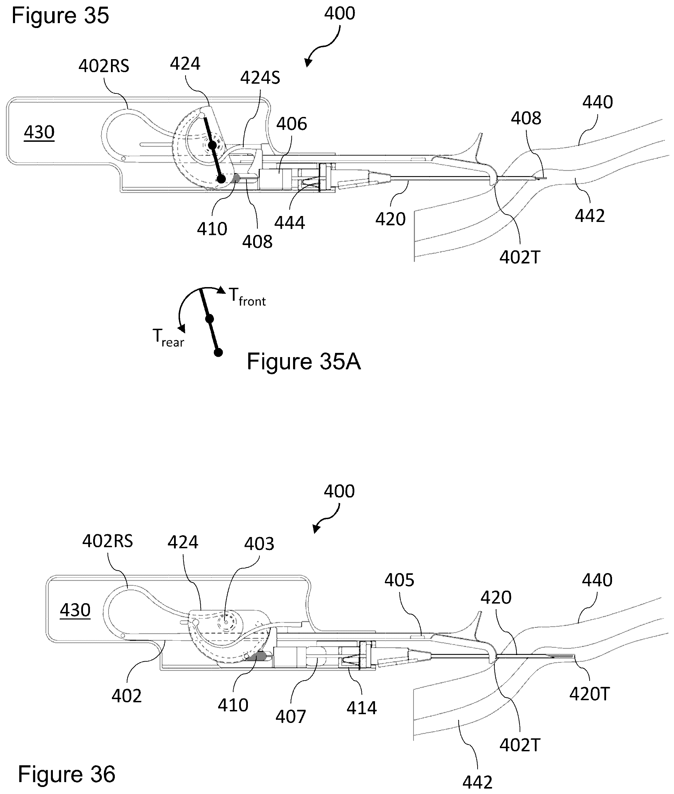

As the needle 407 and probe 408 break through into the lumen of the vein 442, as can be seen in Figure 35, the blunt proximal end 408T of the probe 408 is no longer resisted by the tissue 441 and the probe force (F_{probe}) reduces. As represented in Figure 35A, Trear is now greater than T_{front} and the cam plates 422, 424 rotate together anticlockwise (in this view). The shape of the front springs 422S, 424S wrapped around the associated cam plates 422, 424 is such that as the cam plates rotate, the needle carrier 406 moves rearwards in relation to the handle 430 (which is connected to the catheter 420 via the combination of the slider 424, and clip 444 etc.). The camming surfaces 422CS, 424CS push the probe 408 forwards, the radius of the camming surfaces 422CS, 424CS acting on the pins 461 being greater than the radius of the associated front spring 422S, 424S. The cam plates 422, 424 therefore act as a "virtual lever", in a manner equivalent to the operation of the control mechanisms of the first and second embodiments, so that the probe 408 moves forward as the needle 407 moves backwards relative to the catheter 420. The shape of the front springs 422S, 424S around their associated cam plates 422, 424 defines the rate of movement of the mechanism in this virtual lever. In this embodiment, the shape of the front springs 422S, 424S is arranged so the probe 408 moves forwards into the lumen of the vein 442 relatively fast just after breakthrough into the lumen.

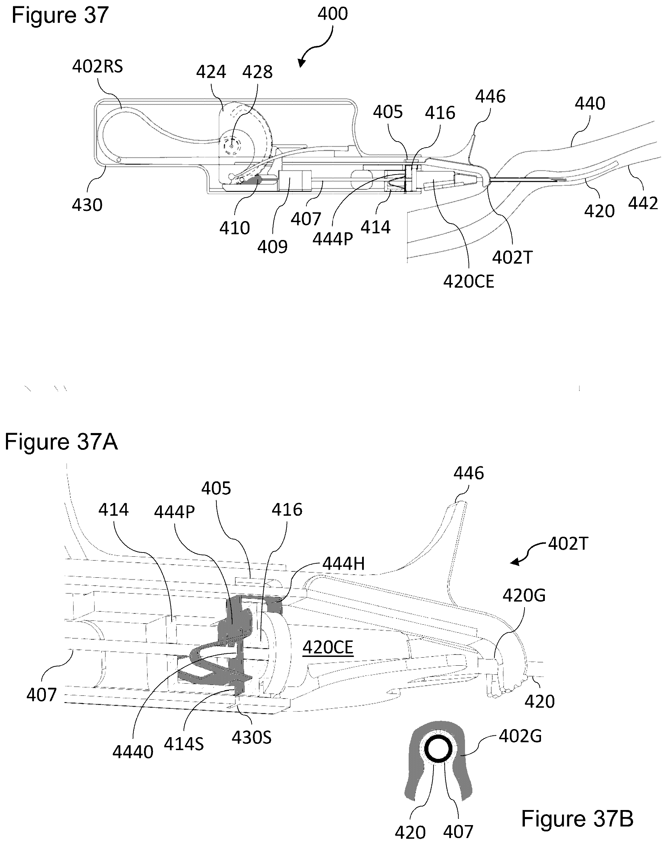

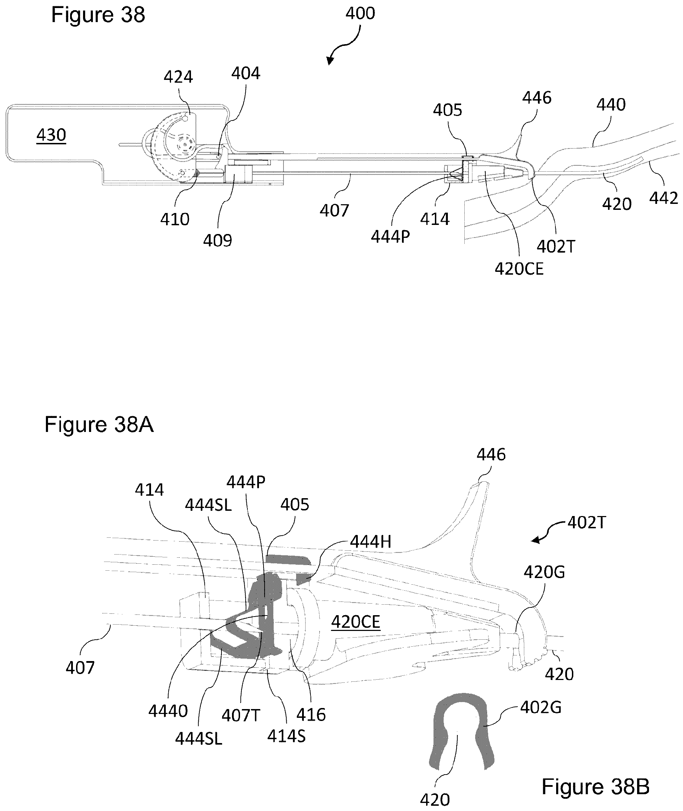

As shown in Figure 36, handle 430 continues to advance along the rail 402. The rear spring 402RS extends rearwards increasing Trear which balances the T_{front} by the extension of the front springs 422S, 424S. This means the needle 407 continues to advance but more slowly than the catheter 420. In this stage of operation, the effective radius of the front springs 422S, 424S increases and the probe boss 403 is now running along a section of the cam plates 422, 424 with a constant radius. The catheter 420 therefore "catches up" with probe 408 and then advances beyond the probe. This operation has the advantage that the flexible catheter tube 420 leads the probe 408 during the later stages of the advance, reducing the risk of snagging on the inside wall of the vein or venous features such as vascular valves etc. As shown in Figure 37, in the next stage of operation the handle 430 continues to its forward end position on the rail 402. The clip 444 moves to Position B as illustrated in more detail in Figure 37A) which prevents the slider 414 from moving forwards or rearwards relative to the notch 405 in rail 402 and also disconnects the handle/casing 430 from the slider 414. It will be seen that in Position B the upper surface of the clip 444 springs up and engages with the notch 405 in the rail 402, preventing the slider 414 from moving forwards or rearwards along the rail 402. A lower edge of the plate 444P defining the hole 444O now rests on the lower surface of the needle 407 which prevents the clip 444 from springing further upwards. The upper hook of the clip still prevents the catheter connection end 420CE from moving forwards off the slider boss 416, the two elements remaining connected. Finally, in the movements of Position B, the plate 444P which extends downwards through the slot 414S in the slider has moved upwards and is no longer engaged in the corresponding slot 430S in the handle/casing 430 so that the handle/casing can therefore move rearwards relative to the slider 414 to retract the needle 407 manually. It will be noted that the device of this embodiment does not have the automated needle retract function of previous embodiments, the needle 407 being manually retracted. The catheter 420 is, in this condition, fully inserted into the vein 442 and the needle 407 has advanced about half the distance of the catheter. Although not shown, probe 408 extends from the lumen of the hollow needle 407 but does not extend beyond the catheter. The user's other hand would, in use, then hold stationary an upstanding tag 446 at the front section of the rail 402 (shown in Figure 38) and quickly slide the handle 430 rearwards to retract the needle 407. This action rotates the cam plates 422, 424 in a clockwise (as viewed in Figure 37) direction which has the effect of also retracting the probe 408 within the retracted needle 407. During this probe retraction, the axle pin 428 will eventually hit the rear end of the slot 430S in the handle 430.

The handle 430 continues to be moved rearwards by the user forcing the needle carrier 406 further backwards over the rear spring section 402RS as shown in Figure 38. The sharp tip 407 of the needle 407 retracts beyond the clip 444 and the clip moves to Position C as shown in Figure 38A and 38B. In the condition or stage of operation of this Position C the needle 407 has now been fully retracted into the slider 414. The clip plate 444P can now move upwards past the tip of the needle 407 and the upper surface of the clip moves fully into the notch 405. The slider 414 cannot now move forwards or backwards relative to the notch 405. The hole 444O defined by the clip plate 444P is no longer aligned with the needle hole in the boss 416 and the plate 444P prevents the retracted needle 407 from moving forwards. The needle 407 is therefore "safe" within the slider 414. At this point the upper hook 444H of the clip is released from the catheter connection end 420CE allowing the entire device 400 to be slid backwards. The catheter 420 disengages the boss 416 and the forward section of the rail 402 can be unclipped from the catheter 420. It will be noted that the shape of the groove 402G in the forward section, shown in more detail in Figure 38B is such that the catheter 420 can only be unclipped from the device 400 when the needle 407 is retracted. The movements described above in relation to Position C for the clip 444 coincide with the stop pin 404 butting up against the needle carrier 406. As described above, the clip 444 prevents the needle 407 moving forwards and the stop pin 404 prevents the needle 407 moving rearwards. Accordingly, the needle 407 is now "safe" with its tip 407T protected within the slider 414 avoiding secondary needle-stick type injury to the user. The device including the needle 407 can now be safely disposed of. It will be appreciated that the front springs with varying effective radii used in this embodiment enable the probe to move quickly during breakthrough into the vein and then slow down relative to the catheter. This results in a probe that does not significantly overshoot the catheter in the end position. As mentioned above, creating the virtual lever from flexible resilient plastic springs gives a particularly smooth action compared to using gear or pin linked components. The simplified features of the mechanism of the semi-automatic control means reduces the associated production cost of the device of this embodiment. Devices in accordance with this embodiment may therefore be easier and cheaper to manufacture and assemble.

### A further device (part of an invention to which the present European patent / application relates)

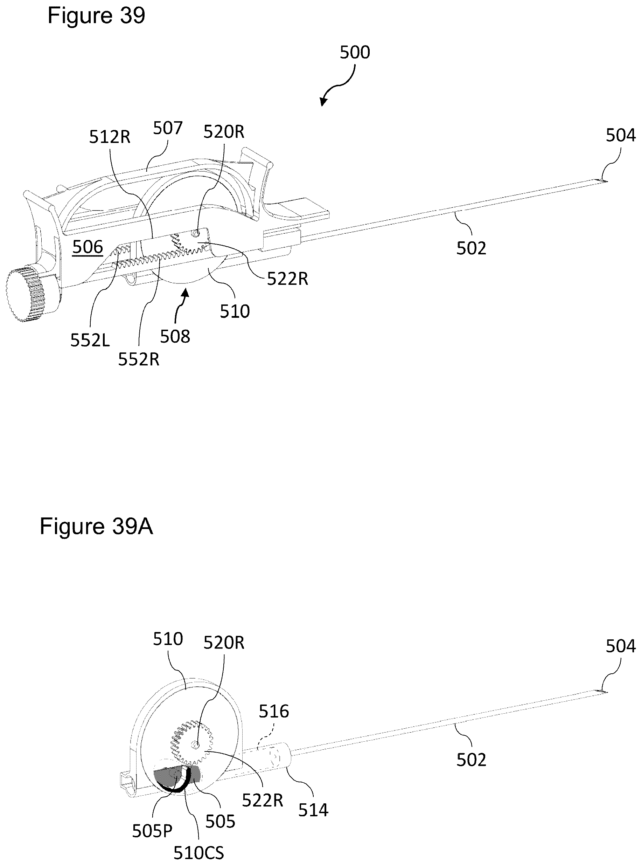

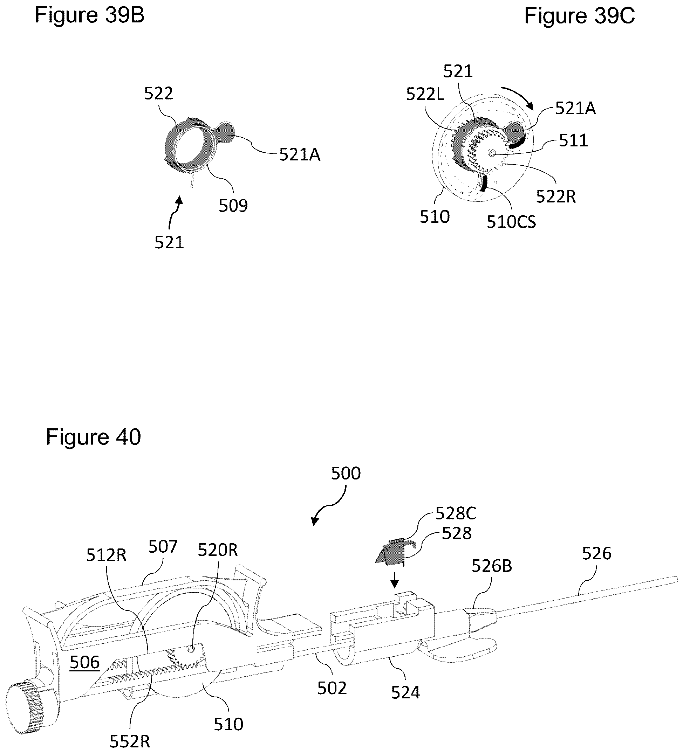

A device 500 in accordance with a fourth embodiment is shown in Figures 39 to 52. This device has the same key components as previous embodiments i.e., a needle, a probe, a tube (e.g., a catheter), a support leg (datum), and a mechanism (e.g., a drive mechanism) to automatically control the relative motions of these components up to, during and after breakthrough into the lumen of a vein based on a "virtual/effective" lever linking the components. There is no "trigger mechanism" as used in certain known device designs - resulting in a smooth action on entering the body cavity such as the vein referred to in this embodiment. A device in accordance with this embodiment uses a support leg in the form of a flexible datum which makes the device more compact and may use less material in manufacture. Again, in this embodiment, a coupling element in the form of a single spring steel clip is used that provides a number of functions in terms of controlling the mechanism and enabling secondary needlestick safety. A device in accordance with this embodiment uses mechanical linkages in the form of rack and pinion gears, in place of the tapes or springs used in some previous embodiments, which may be easier to manufacture. This embodiment replaces the probe spool of earlier embodiments with a single gear tooth or cam acting on a face of an element such as an over-moulding connected to the probe which functions to enable the catheter to "catch up" and "overtake" the probe in use. Devices in accordance with this embodiment may be more compact and easier to manufacture. They may also be more reliable in use.

The catheter device 500, which is shown partially complete in Figure 39, comprises a needle 502 and probe 504 (the tip of which is just visible within the needle tip) which slides within the needle 502 and a "skeletonised" body 506 which forms a user-grippable handle 507 and supports a control mechanism (or drive mechanism) generally designated as 508. The control mechanism 508 includes a rotatable element in the form of a wheel 510 the axle of which runs along tracks 512L and 512R formed by the body 506. The wheel 510 has a spur gear 522R which engages in a gear rack 552R formed as part of the body 506. The gear 522R is mirrored on the left-hand side 522L and engages with a gear rack 552L formed on the left-hand side of the body 506.

As shown in Figure 39A, the proximal end of the needle 502 is connected to a needle holder 514. The proximal end of the probe 504 has an over-moulding 505 that slides within a cylinder 516 of the needle holder 514. The wheel 510 and associated gears 522 are free to rotate within the needle holder. The external rim of the wheel 510 is shaped so that one end is formed into a camming surface 510CS similar to a single gear tooth acting against a face incorporated into the moulding 505. As the wheel 510 rotates anticlockwise (as viewed) it pushes the probe 504 distally relative to the needle 502. A pin 505P on the moulding 505 acts on the inside of the external rim to pull the probe 504 proximally when gear B rotates clockwise (as viewed). Whilst the moulding 505 is a snug fit within the cylinder 516 of the needle holder 514 such that it can slide freely, blood (or other fluid) entering the cylinder chamber from the proximal end of the needle is contained within this chamber. The cylinder 516 therefore acts as a flash chamber.

Pins 520L and 520R protrude from the left and right of gears 522L and 522R respectively and run against corresponding tracks 512L and 512R formed in the handle 507. These pins position the gears 522 at the correct height relative to the gear racks 552 and so prevent the assembly from pushing upwards. The wheel 510 can therefore roll forwards and backwards along the racks 552L and 552R.

The control gear 521 (shown in Fig 39B) consists of a spur gear arranged to rotate around a hub of wheel 510 and also has an off-centre arm 521A. One leg of a wire torsion spring 509 engages with the off-centre arm. As shown in Figure 39C, the wheel 510 has a central axle 511 on which the hub of the control gear rotates. The wheel 510 also forms identical spur gears 522 (right spur gear 522 R visible, left spur gear 522L just visible behind the control gear 521) on each end of the axle of the wheel 510. The axle of the wheel 510 is connected to an external rim 510CS which forms end stops for the off-centre arm 521A of the control gear 521 as shown in the cutaway in Figure 39C. The other leg of the torsion spring acts against one of these stops such that the control gear 521 is urged in the direction of the arrow in Figure 39C relative to the wheel 510.

As shown in Figure 40, the needle 502 passes through a central boss formed by a slider component 524. The catheter 526 also slides over the needle 502 and the catheter body 526B butts up against the slider. A spring steel clip 528 locates within the slider 524 and connects the slider to the catheter 526 and acts as described with reference to the following drawings. A tag 528C protrudes upwards from the spring steel clip 528.

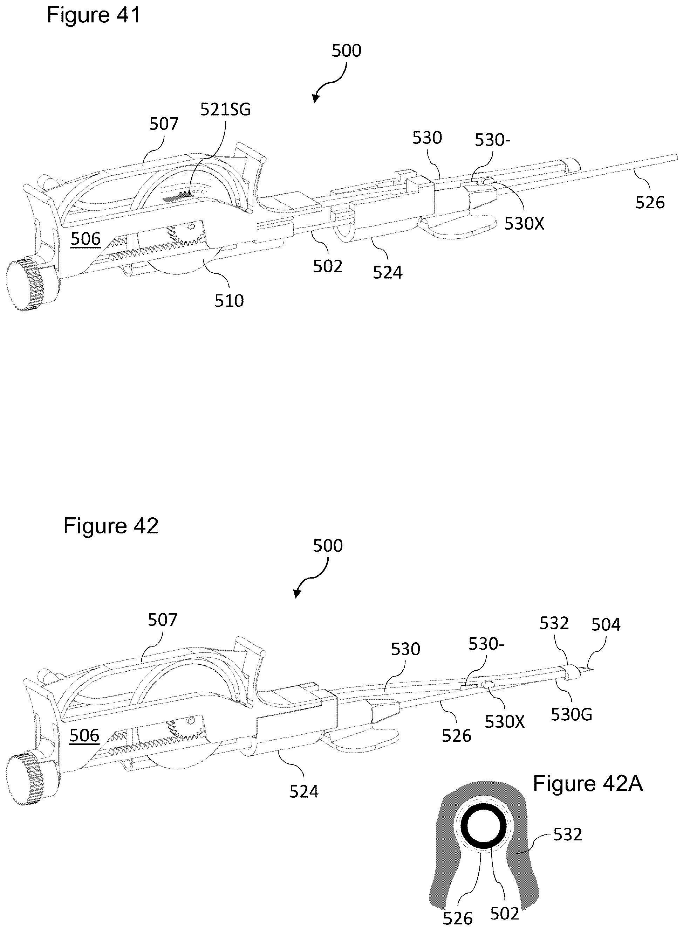

As shown in Figure 41, a flexible datum component 530 having a lower toothed surface is inserted into a track formed within the handle 507. The track guides the flexible datum 530 through the handle 507, over the top of the control gear 521, so that it exits at the rear of the handle. The cutaway in the wheel 510 in Figure 41 shows gear teeth along the lower surface of the flexible datum 530 engaging with the spur gear 521SG teeth of the control gear 521 similar to a rack and pinion. The forward part of the flexible datum 530 passes through the slider 524 component and extends along the catheter. A groove 530G running along the underside of the datum component 530 sits over the top of the tag formed by the spring steel clip. A slot 530- is formed in the side of the datum component 530. End stops 530X protrude from each side of the datum component 530.

As shown in Figure 42, to assemble the device for use the slider 524 is pushed rearwards and grooves in the slider engage with shoulders formed in the handle 507.

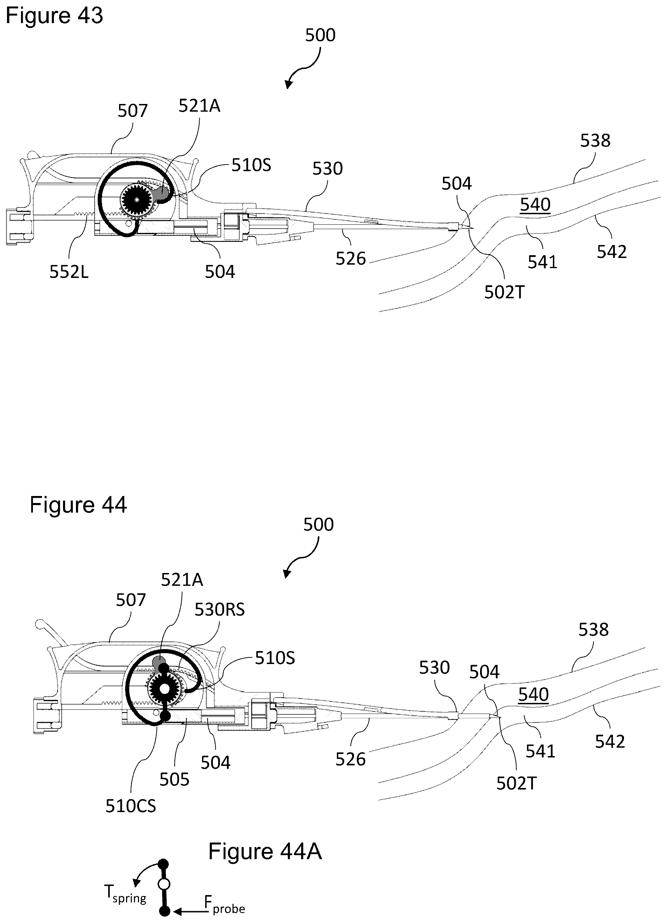

A clip 532 at the distal end of the datum 530 push fits over the catheter 526 as seen in cross-section in Figure 42A. The catheter 526 sits within the groove 530G in the underside of the datum 530. This clip 532 is designed to butt up against the skin of the patient, serving as a datum for the device. The thus assembled device 500 is ready for use. The sequence of steps to insert a catheter into a patient's vein using the device 500 will now be described referring to Figures 43 to 52.

In the initial condition for the device 500 shown in Figure 43, the control gear arm 521A sits against the stop 510S formed in the wheel 510. As the needle tip 502T enters the tissue 540 of the patient, the probe 504 is aligned with the end of the needle 502. The end of the datum 530 butts up against the skin 538 of the patient.

Figure 44 shows the advance of the needle, probe, and the catheter through tissue 540. The handle 507 is pushed forwards and the catheter 526, needle 502 and probe 504 advance together through the tissue 540 of the patient. The datum 530 remains butted against the skin 538 of the patient, so the flexible toothed rear section 530RS of the datum moves rearwards through the handle 507. This rotates the control gear 521 anti-clockwise (as viewed), lifting the arm 521A away from the stop and increasing the opposing force in the torsion spring 509 (not shown). This urges the wheel 510 in an anticlockwise direction, but it cannot rotate because the distal probe end 504 is engaged with the tissue 540 and the probe transmits a force through the moulding 505 onto the camming surface 510CS of the wheel 510 to prevent rotation. The wheel is therefore in equilibrium as shown in the free body diagram Figure 44A. The catheter 526, needle 502 and probe 504 therefore advance together through the tissue 540.

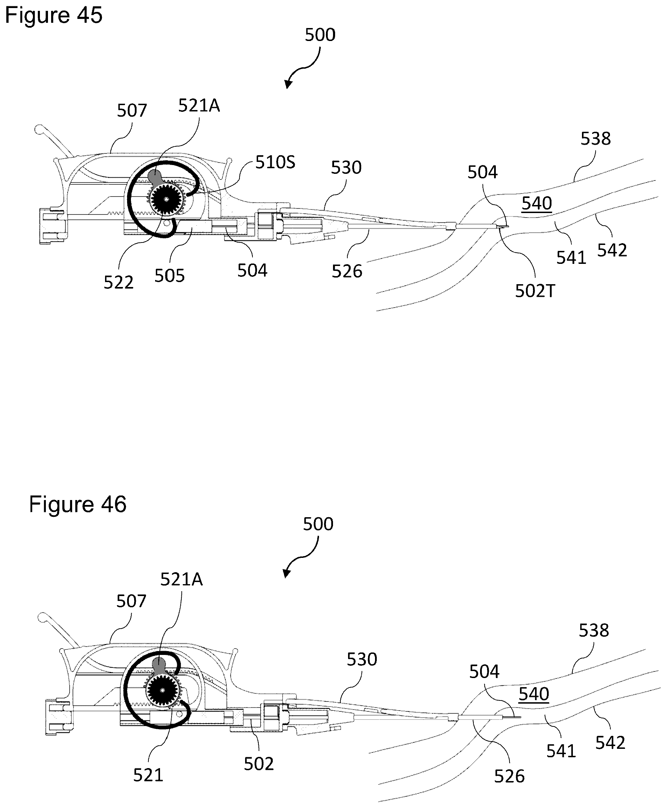

Figure 45 illustrates the "breakthrough" stage of use. As the needle 502 breaks through into the lumen 541 of the vein 542, the opposing force from the tissue 540 onto the end of the probe 504 reduces. The torsion spring 509 now rotates the wheel 510 anticlockwise (as viewed) pushing the probe 504 forward relative to the needle 502. As the wheel 510 rotates, the needle holder 514 moves rearwards relative to the handle 507 due to the rack and pinion formed by the gear teeth along the lower surface of the flexible datum 530 engaging with the spur gear 521SG teeth. The effective radius of the camming surface 510CS of the wheel 510 (pushing the probe moulding 505) is greater than the radius of the spur gear 522 (engaged with the rack 552 on the handle 507).

This difference in effective lever lengths controls the motions of the needle 502 and probe 504 relative to the catheter 526. As the probe 504 advances along the lumen, the needle 502 retracts into the catheter.

Figure 46 illustrates the stage of the advance into vein in which the handle 507 continues to be advanced by the user pushing the catheter 526 further into the vein 542. The elongate datum 530 continues to move rearwards through the handle 507, rotating the control gear 521 anticlockwise (as viewed). The torsion spring 509 continues to rotate the wheel 510 so that the arm 521A of the control gear starts to return to the end stop of the rim. Thus, the needle 502 continues to move rearward relative to catheter 526 while the probe 504 moves forward relative to the catheter 526.

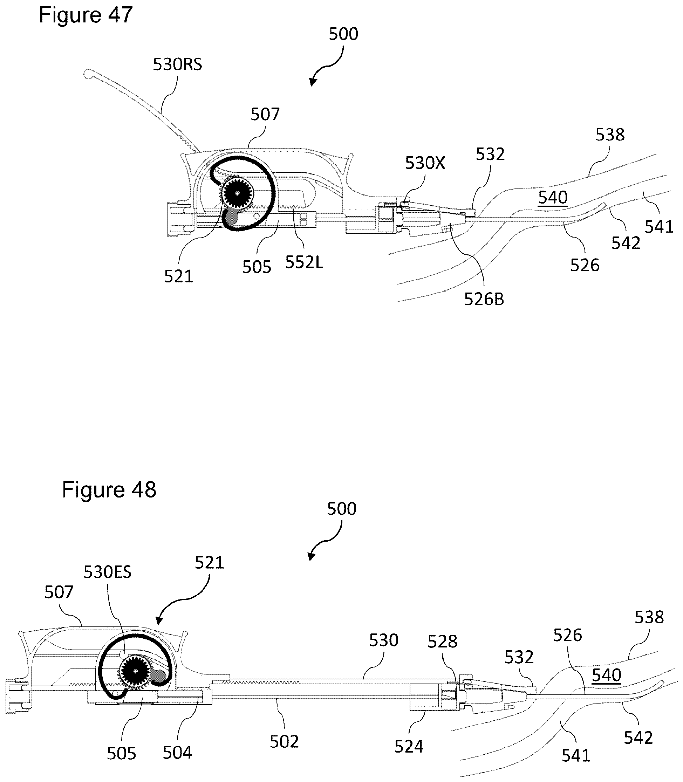

Figure 47 illustrates the end position with the catheter 526 fully advanced into the lumen 541 of the vein 542. While the needle 502 has continued to retract relative to the catheter 526 as the gear 522 rolls backwards along the rack 552, the probe moulding 505 is now running along a section of the rim of the wheel 510 with constant radius so is no longer advanced relative to the needle 502. This means that the catheter 526 "overtakes" the probe 524 towards the end of the insertion and advancement stage. The arm 521A of the control gear 521 has reached the end stop of the wheel 510. The datum 530 has passed fully rearward through the handle 507 such that the end stops 530X formed in the datum engage in grooves formed in the slider which prevent the datum from moving further rearwards. The slot 530- in the groove of the datum 530 is now aligned with the tag 528C on the spring steel clip 528. The action of the spring steel clip 528 engages the slider 524 with the datum 530 at this point so that it cannot move rearwards, as described in more detail in the later drawings. The clip 532 on the end of the datum 530 has also been forced to unclip from the catheter 526 by the wedge-shaped end of the catheter body 526B pushing it upwards.

Figure 48 illustrates the "retract" stage. As the catheter 526 and end of the datum 530 are held stationary relative to the patient, the handle 507 is moved quickly rearwards.

The slider 524 is now connected to the datum 530 at this point and slides off the handle 507 as it moves rearwards. As the handle 507 moves rearwards, the flexible elongate datum 530 passes through the handle 507. This rotates the control gear 521 and wheel 510 clockwise (as viewed) which retracts the probe 504 back within the needle 502. At this point the elongate flexible datum 530 reaches the last tooth of the control gear 521 and the tail of the datum passes over a smooth section of the control gear. The handle 507 continues to move rearwards until an end stop 530ES on the datum 530 butts up against the control gear 521. At this point the needle 502 has retracted fully behind the spring steel clip 528 within the slider 524 and the spring steel clip has acted to prevent the needle 502 moving forwards relative to the slider as described in more detail later. The needle tip is now safe within the slider 524 which is held in place by the fully extended datum 530.

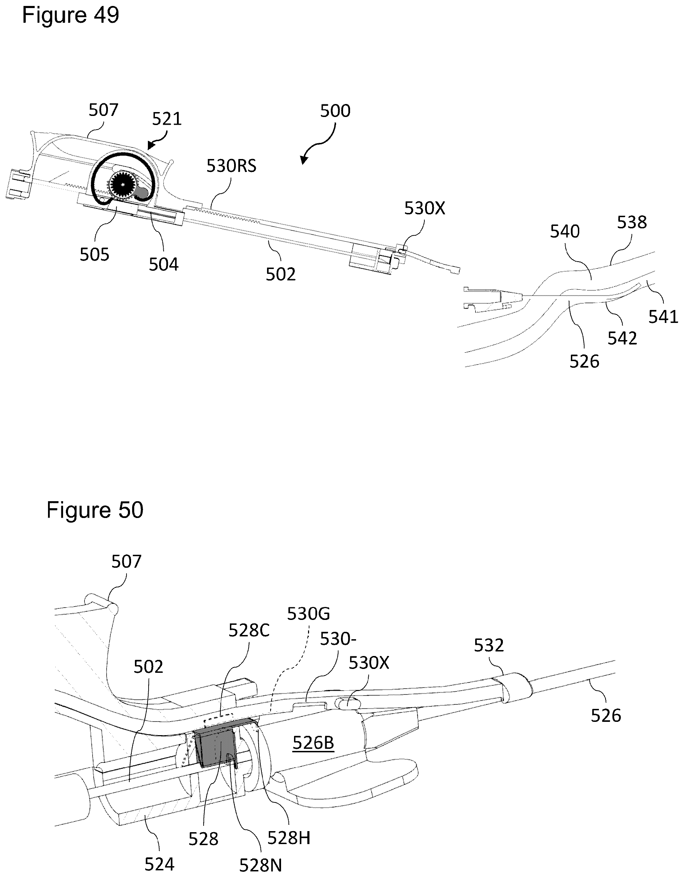

Figure 49 illustrates the removal step. Once the needle 502 passes rearwards behind the spring steel clip 528, the clip acts to release the catheter body from the slider 524.

The remainder of the device 500 can now be removed from the catheter 526 and disposed of safely.

The operation of the slider 524 and clip 528 in the above sequence will now be described in more detail with reference to Figures 50 to 52. As described above slider 524 contains a clip 528 made from a spring steel pressing. The clip 528 is formed to have a plate 528P with a notch 528N in it through which the needle 502 passes. The clip 528 has "Z" shaped spring legs that exert a sideways force urging the plate 528P to the right. The plate 528P bends over to form a hook that connects over the rim of the catheter body 526B.

The tag 528C extends upwards from this hook.

The spring clip 528 has three stages of operation as follows:
Clip Position A is shown in Figure 50 and occurs while the slider 524 moves from its initial position up to the slot in the datum groove 530G. In this position, the tag 528C of the clip is running within the groove 530G formed in the lower side of the datum. This is preventing the clip from springing to the right. The notch 528N in the plate 528P is therefore in clearance of the needle 502 allowing the needle to slide completely smoothly without touching the notch. The hook 528H of the clip prevents the catheter body 526B from moving forwards off the boss of the slider 524. The two components are therefore connected.

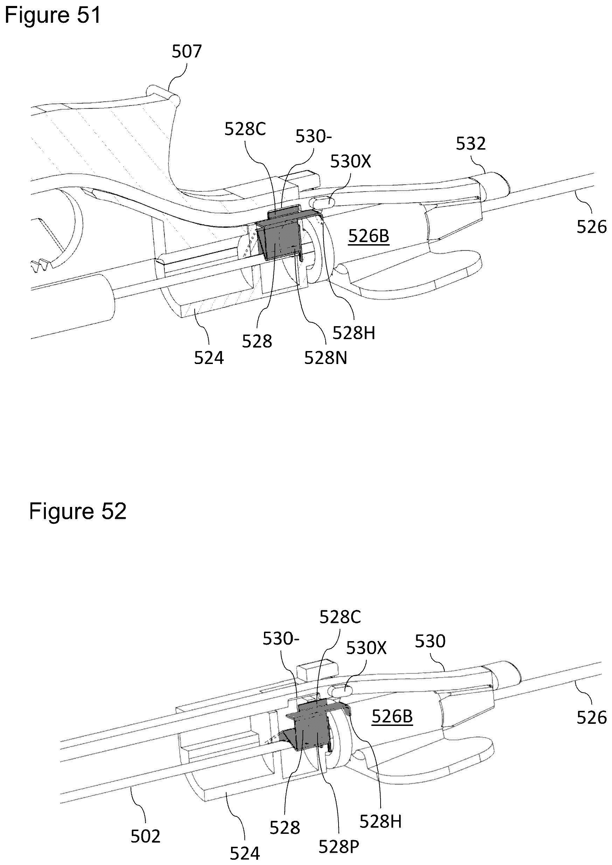

Figure 51 shows Clip Position B which is taken when the slider 524 is in the end position. In this position, the stops 530X on the datum 530 engage with grooves in the slider 524. The tag 528C is aligned with the slot 530- which extends transverse to the datum groove 530G so the plate 528P can now move to the right such that the notch 528N in the plate is now resting against the needle 502. This small movement of the clip 528 means that the tag 528C is now within the transverse slot 530- of the datum 530 and thus prevents the slider 524 from moving forwards or rearwards along the datum 520. The hook 528H of the clip 528 still prevents the catheter body 526B from moving forwards off the boss of the slider 524. The two components remain connected.

Figure 52 illustrates clip position C - with the slider in the end position.

The needle 502 has now been fully retracted into the slider 524. The plate 528P can now move completely to the right, past the tip of the needle 502. The notch 528N is no longer aligned with the needle hole in the boss and the plate 528P prevents the needle 502 from moving forwards. The stops 530X prevent the slider 524 from moving forwards relative to the datum 530. The needle 502 is therefore "safe" within the slider 524. The hook of the clip 528 is released from the catheter body 526B allowing the entire device 500 to be slid backwards, disengaging the 526 from the boss.

As noted above, a device in accordance with this embodiment may have several advantages. As there is no "trigger mechanism as used in certain known device designs it has a smooth action on entering the vein. The flexible datum makes the device more compact and may use less material in manufacture. The use of rack and pinion gears, in place of the tapes or springs used in some previous embodiments may be easier to manufacture. A device in accordance with this embodiment hey may also be more reliable in use.

### Another device (part of an invention to which the present European patent / application relates)

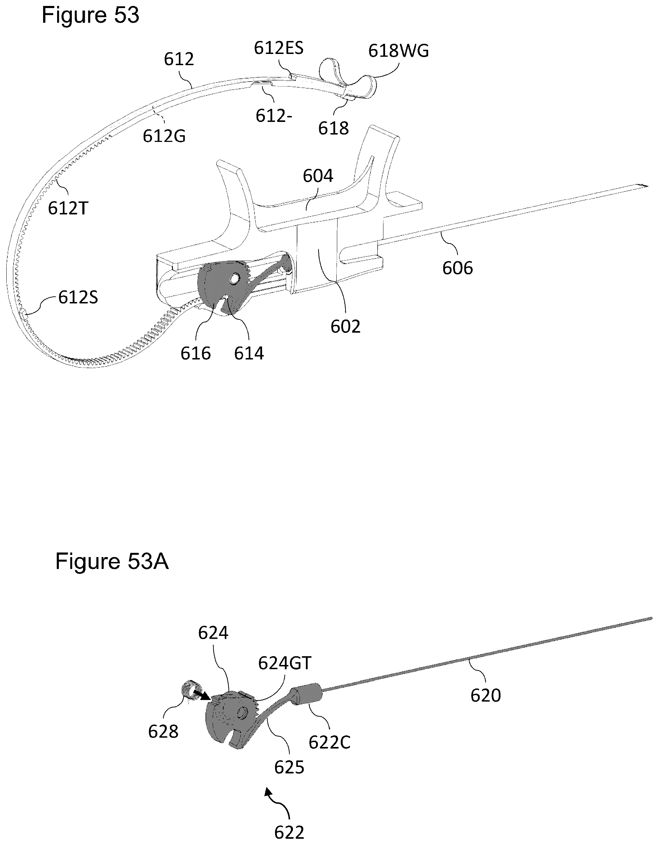

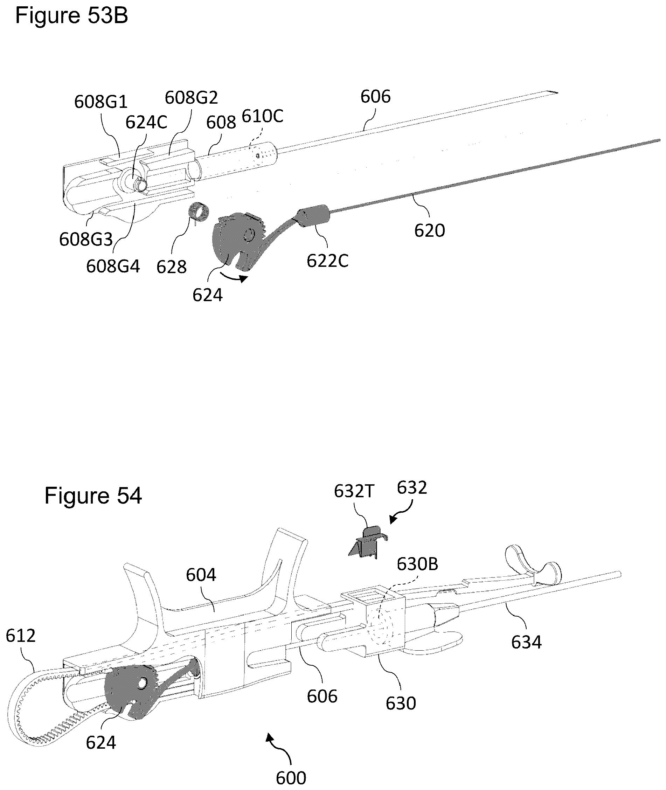

Another device 600 is shown in Figures 53 to 63 and generally speaking has the same key components as previous embodiments: a needle, a probe, a tube (e.g., a catheter), a support leg (e.g., a datum), and a mechanism (e.g., a drive mechanism) to automatically control the relative motions of these components before, during and after breakthrough into the lumen of the vein based on an effective lever linking the components. Unlike some known devices, such as that described in US5330432, there is no trigger mechanism - which results in a smoother action on entering the body cavity i.e., in case a vein. This embodiment also uses a support leg in the form of a flexible datum which makes the device more compact and uses less plastics material in manufacture. Furthermore, this embodiment also uses mechanical linkages in the form of rack and pinion gears in place of the tapes or springs used in some previous embodiments, and so may be easier to manufacture. The main difference between this embodiment and the previous embodiment is that on breakthrough into the lumen the needle distally advances a distance approximately five times the diameter of the needle and then stops relative to the datum. The catheter continues to extend into the lumen. This allows the needle to advance sufficiently to support the relevant section of the catheter in the region where it is surrounded by tissue, but the needle does not continue unnecessarily into the lumen of the vein where the route may become more curved or tortuous. In contrast, in previous embodiments, the needle continues to advance after breakthrough into the lumen but at a slower rate than the catheter.

As shown in detail in Figure 53, a main body 602 forms a handle component 604. The handle is above a needle 606 and a cylinder 610 of a needle housing 608. The handle 604 forms forward and rear grips for the thumb and forefinger of a user. A flexible elongate datum 612, which forms a toothed track 612T, extends rearwards from the handle 604 as shown. The teeth of the track 612T are moulded into a section of the track surface towards one end of the datum 612. Further along the datum 612 an intermediate stopper 612S extends laterally from the side of the track 612T. In the section of the datum track after the gear teeth, a groove 612G is formed in the underside of the track. A longitudinal slot 612- is formed in a section of this groove. A further end stop 612ES is formed beyond the slot 612-. A wing-shaped grip 618WG is formed at the end of the datum 612. A control pin 614 extends laterally from the side of the track 612 and engages with a cam surface provided by the control wheel 616.

The probe 620, which is shown in more detail in Figure 53A, consists of a flexible coiled wire as in previous embodiments. A plastic injection moulded component 622 is over-moulded onto the proximal end of the coiled wire probe 620. This component 622 consists of a cylindrical piston section 622C, and a control wheel 624 which are interconnected by a link arm 625. The control wheel 624 is formed to provide gear teeth 624GT (as shown in Figure 53A). The elongate link arm 625 which connects the control wheel 624 and cylinder section 622C is relatively thick but tapers to a very thin section at each end to create a flexible joint. A torsion spring 628 is housed in the hub of the control wheel 624.

As shown in Figure 53B, the probe 620 is housed in the needle holder 608 which is over-moulded on a sharp needle 606 which projects forward. The probe 620 slides freely within the needle 606. The needle holder 608 forms a cylinder 610C in which the piston 622C also slides. The control wheel 624 is mounted on an axle 624C protruding from the needle holder 608. The control wheel 624, which can rotate about the axle 624C and the torsion spring 628, urges the control wheel in the direction indicated by the arrow. Guides and tracks 608G1,608G2, 608G3 and 608G4 are formed by the needle holder 608

As shown in Figure 54, the main body 602 slides over the needle housing 608. The flexible datum 612 is threaded into the mechanism such that it fits in the guides and tracks 608G1, 608G2, 608G3 and 608G4 formed by the needle holder 608 and also a guide in the main body 602. The teeth of the track 612T now sit directly over the control wheel 624 mounted on the needle holder 608. The forward part of the datum track 612T now runs forward above the needle 606. Figure 54 also shows how the needle 606 passes through a central boss 630B within a slider component 630. A spring steel clip 632 locates within the slider 630 (although it is shown detached from the slider in Figure 54) and connects the slider to the catheter 632 and acts generally as described above in relation to the previous embodiment. A tag 632T protruding upwards from the spring steel clip 632 and locates in the groove 612G on the lower surface of the flexible datum track 612T.

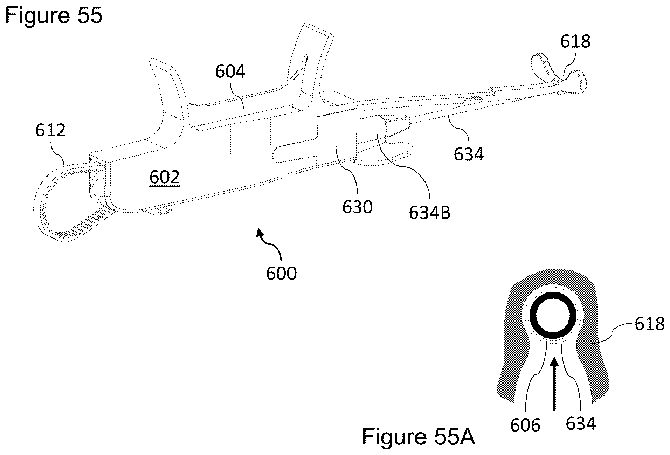

Figure 55, which shows a fully assembled device. A datum grip 618 formed at the distal end of the flexible datum 612 is a push fit over the catheter 634. Figure 55A shows in cross section how the catheter is held by the datum grip 618.

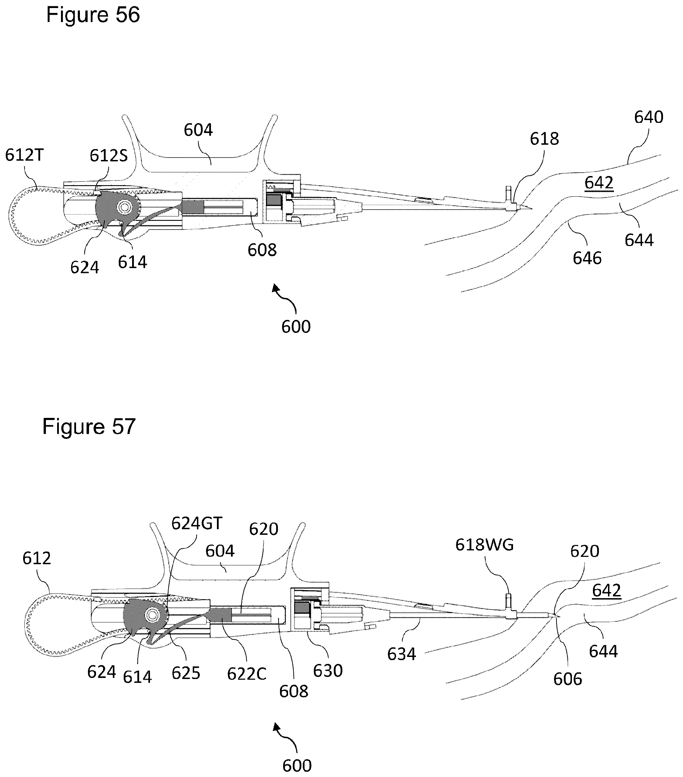

In the "initial" condition, or stage of operation, shown in Figure 56, the torsion spring 628 urges the control wheel 624 anticlockwise as viewed. This rotation is prevented by the intermediate stopper 612S on the flexible datum track 612T sitting in a notch formed within the control wheel. The control pin 614 on the flexible track 612T is engaged in the cam surface formed in the control wheel 624. The datum grip 618 at the distal end of the datum 612 butts up against the skin 640 of the patient.

The "advance" stage through the tissue 642 of the patient is shown in Figure 57. The needle 606's tip enters the tissue 642 of the patient, the probe 620 being aligned with the end of the needle. The handle 604 is pushed forwards and the catheter 634, needle 606, and probe 620 advance together through the tissue 642 of the patient. The datum 612 remains butted against the skin 640 of the patient, so the flexible track section 612T of the datum moves rearwards through the upper guides of the slider 630, handle 604, and needle holder 608. Thus, the intermediate stopper 612S moves rearwards and disengages the notch in the control wheel 624. The control wheel 624 is now free to rotate anticlockwise (as viewed). However, the control wheel 624 cannot rotate because the probe 620's end is engaged with the tissue/skin 640 and 642 and the probe transmits a force through the piston 622C and the link arm 625 onto the outer rim of the control wheel 624. The handle 604 is directly connected to the control pin 614 which is engaged with the cam surface. This draws the needle holder 608 along with the handle 604. The catheter 634, needle 606, and probe 620 therefore advance together through the tissue 642.

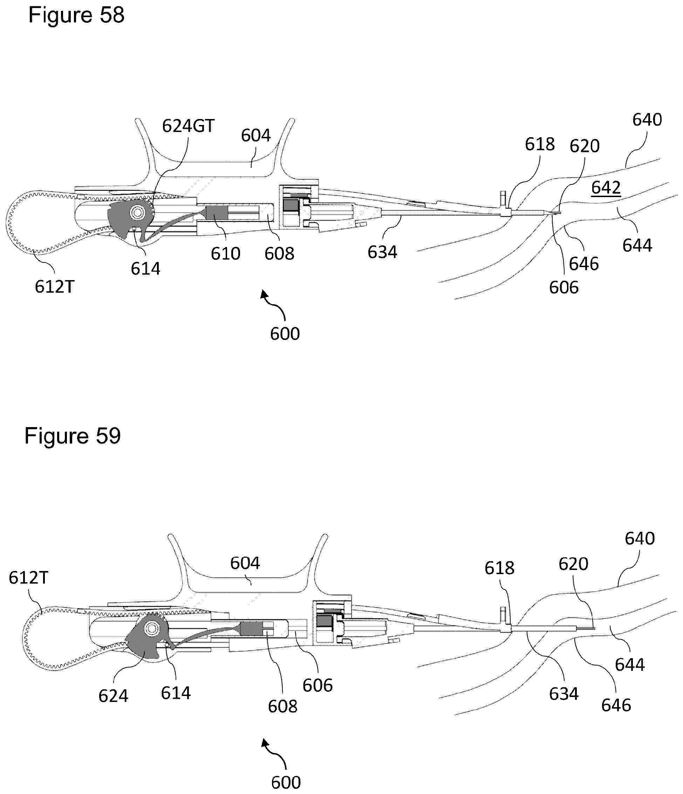

In the "breakthrough" stage (shown in Figure 58), as the needle 606 breaks through into the lumen 644 of the vein 646, the opposing force from the tissue 642 (Fₜᵢₛₛᵤₑ ) onto the end of the probe 620 reduces. The torsion spring 628 (not shown) can now rotate the control wheel 624 anticlockwise (as viewed) pushing the probe 620 forward relative to the needle 606. This protects the wall of the vein 646 from the sharp needle 606. The cam surface is designed so that there is sufficient space to rotate away from the control pin 614 and allows the gear teeth 624GT of the control wheel 624 to rotate upwards to the teeth in the flexible track 612T. The gear teeth 624GT of the control wheel 624 will therefore engage with the next available corresponding tooth in the flexible track 612T depending on how far the needle holder 608 has travelled relative to the datum 612 before the point of breakthrough.

The stage of advancement into the vein is shown in Figure 59. The handle 604 continues to be advanced, pushing the catheter 634 further into the vein 646. The control pin 614 engages once more with the retaining face of the cam surface and the gear teeth are engaged in the flexible track 612T. As the handle 604 now moves forward, the control pin 614 acting on the cam surface rotates the control wheel 624 advancing the needle 606 at about half the rate of the catheter. The needle 606 will be advanced until the control pin 614 is clear of the cam surface after which the handle 604 will continue forwards but the needle 606 will stay stationary relative to the datum track 612T. It can be seen that the relative rate of advance of the catheter 634, probe 620, and needle 606 can be controlled by the design of the control wheel 624 (gear radius, cam surface geometry etc.). The distance that the needle 606 advances after breakthrough can also be controlled through the design of this mechanism. The control mechanism may be designed to advance the needle 608 a distance of approximately four to six, preferably five times, the needle diameter after breakthrough. The distance should be sufficient to support the catheter as it is passing through skin, tissue and the proximal vein wall. It is advantageous that the needle does not continue significantly further than this into a region of the vein that might be more curved or tortuous.

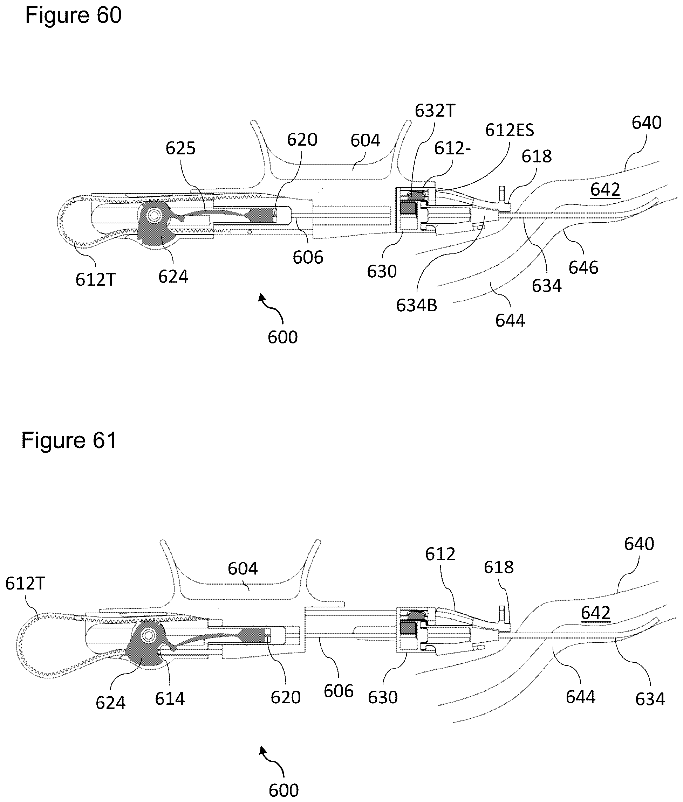

In the "end" position illustrated in Figure 60, the catheter 634 is fully advanced into the vein 646. The control wheel 624 is stopped after rotating about 90° limiting the forward motion of the needle 606. The link arm 625 has pushed the probe 620 to its full forward extent. The geometry is such that after breakthrough, the probe 620 moves faster forward than the catheter 634 but, as the catheter continues its travel, it "overtakes" the probe 620. At this stage the slider 630 has reached the end stop 612ES on the flexible track 612T and can go no further. The slot 612- in the groove of the datum 612 is now aligned with the tag 632T on the spring steel clip 632. The action of the spring steel clip 632 engages the slider 630 with the datum track 612T at this point so that it cannot move rearwards. The datum grip 618 on the end of the datum 612 has also been forced to unclip from the catheter by the wedge-shaped end of the catheter body 634B pushing it upwards. Such a co-operation between the wedge-shaped end of the catheter body, and a detachable component on the datum, such as the datum grip 618, is advantageous,

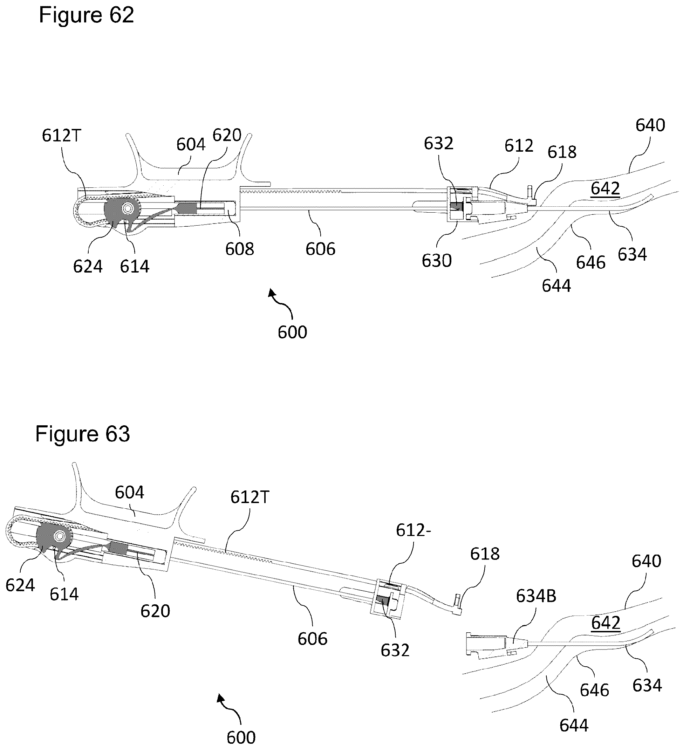

The "retraction" stage is shown in Figure 61 and 62. Figure 61 shows how the catheter 634 and end of the datum 612 are held stationary relative to the patient using the wing-shaped grip 618WG. The handle 604 is moved quickly rearwards. The slider 630 is now connected to the datum 612 at this point and slides off the handle 604 as it moves rearwards. As the handle 604 moves rearwards, the flexible datum track 612T passes through the handle 604. The control pin 614 moving rearwards meets the stationary cam surface of the control wheel 624. This rotates the control wheel 624 clockwise, retracting the probe 620 back within the needle 606 and eventually disengaging the gear teeth of the control wheel with the flexible track 612T.

As shown in Figure 62, the control wheel 624 can now slide rearwards along the track and the handle 604 and needle carrier 608 move rearwards as one. The flexible track 612T is now flush against the rear curvature of the needle holder 608 which can go no further backwards. At this point the needle 606 has retracted fully behind the spring steel clip 632 within the slider 630 and the spring steel clip has acted to prevent the needle 606 moving forwards relative to the slider. In this connection, the steel clip operates generally as described for the clip 528 of the previous embodiment. The tip of the needle 606 is now safe within the slider 630 which is held in place by the fully extended datum 612.

Finally, as shown in Figure 63, once the needle 606 passes behind the spring steel clip 632, it releases the catheter body 634B from the slider. The catheter 634 is now released, and the device can be removed and safely discarded.

The device of this embodiment has several advantages. Again, it will be noted that unlike some known devices, such as that described in US5330432, there is no trigger mechanism in the device of this embodiment, which results in a smoother action on entering the vein. Again, the flexible datum used in this embodiment makes the device more compact and uses less plastics material in manufacture. Furthermore, the use of rack and pinion gears in place of the tapes or springs used in some previous embodiments may result in easier manufacture. The control of the advance of the needle ensures that it does not continue unnecessarily into the lumen of the vein where the route may become more curved or tortuous, thus reducing the risk of damage.

### Further device (part of an invention to which the present European patent / application relates)

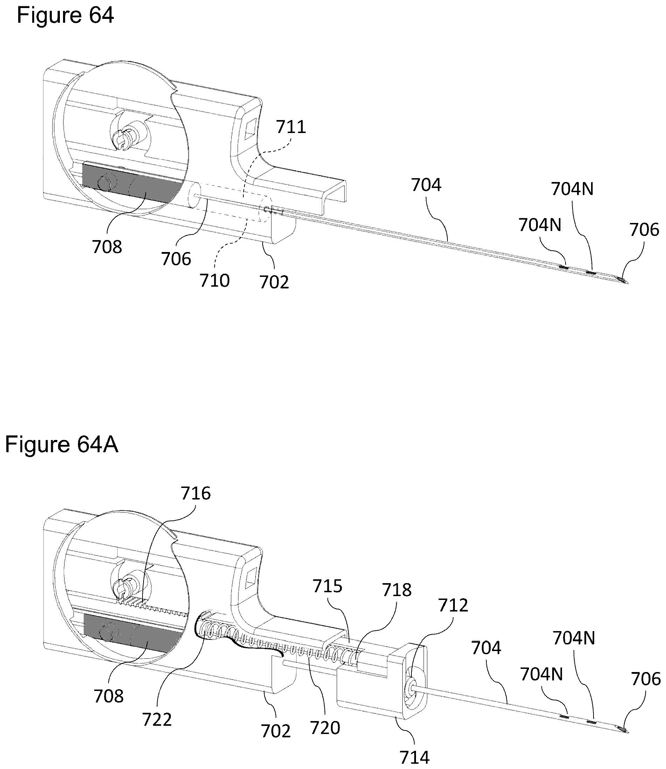

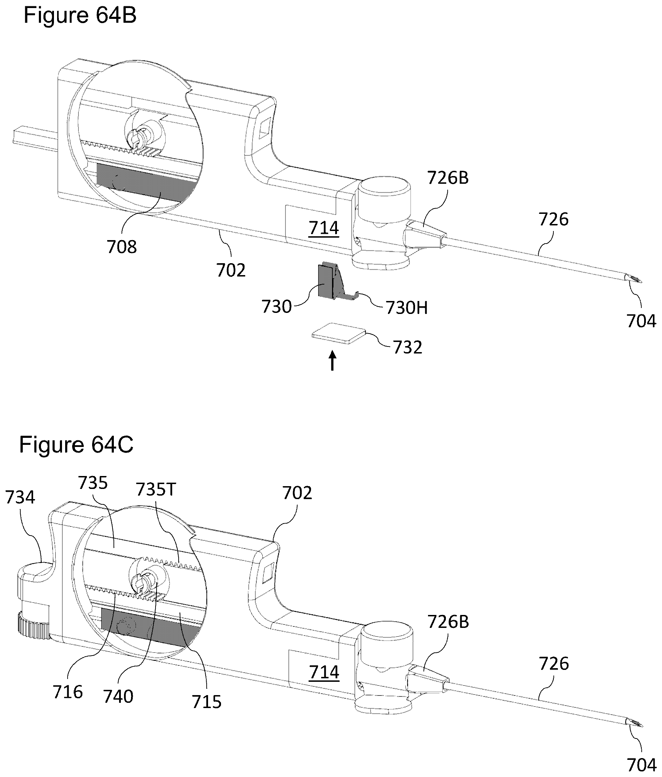

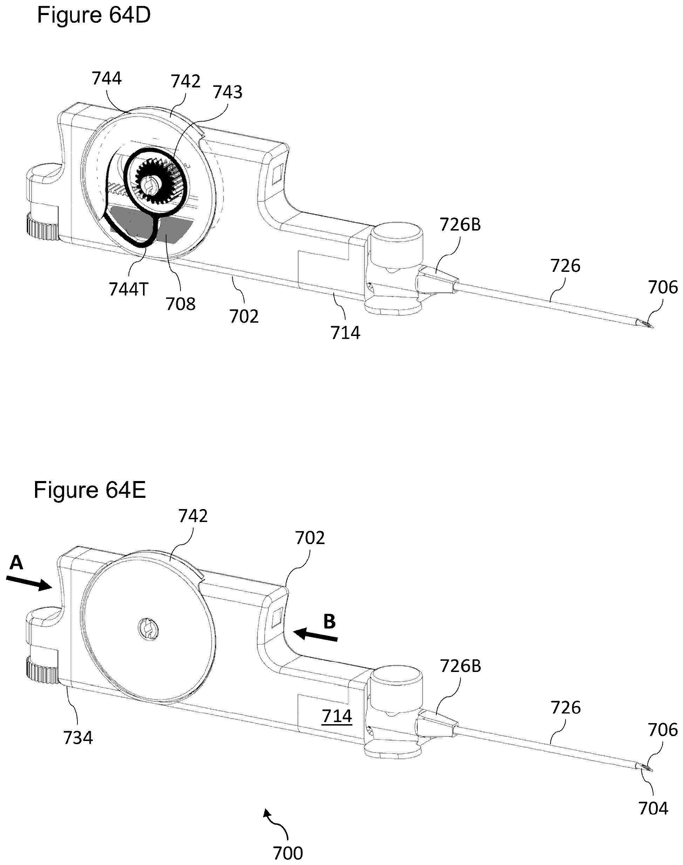

A further device 700 in accordance with this embodiment is shown in Figures 64 to 71. The device 700 has the same key components as in previous embodiments: a needle, a probe, a tube (e.g., a catheter), and a mechanism (e.g., a drive mechanism) to automatically control the relative motions of these components on breakthrough into the lumen of the vein based on an effective lever linking the components. This embodiment does not use a support leg or datum and relies on the user to initiate the mechanism on observation of a blood flash as the needle enters the vein. As such a device in accordance with this embodiment is less "automated" than previous embodiments. However, it can be used one-handed and requires fewer manipulations than a conventional device. Again, unlike some known devices, such as that described in US5330432, there is no trigger mechanism - which results in a smoother action on entering the body cavity i.e., as described below in a vein.

The various components of the device 700, and their interactions, are illustrated in Figure 64 onwards. Figure 64 shows how the needle housing 702, which is an injection moulded component, is connected to a needle 704. A probe 706 can slide within the needle 704. The proximal end of the probe 706 has an over-moulding 708 that slides within a cylinder 710 in the needle holder 702. The over-moulding 708 is a snug fit within the cylinder 710 of the needle holder 702 such that it can slide freely but blood (or other fluid) entering the cylinder chamber 711 from the proximal end of the needle 704 is contained within this chamber. This chamber 711 therefore constitutes a flash chamber. The needle 704 also has notches 704N extending into the lumen of the needle towards its distal end that allow the blood to be viewed through the translucent catheter (not yet shown).

Figure 64A shows how the needle 704 passes through a boss 712 within a slider component 714. The slider 714 consists of a rearward extending arm 715 that slides within a groove formed within the needle housing 702. The rearward facing arm 715 has a gear rack 716 formed in its upper surface. The slider 714 is also formed to provide a spigot 718 over which a compression spring 720 loosely fits. The other end of the spring 720 fits over a second spigot 722 formed in the needle holder 702. The cutaway section shows the compression spring 720 within the needle holder 702. The compression spring 720 is a tighter fit over spigot 722 such that, in later stages of deployment, the spring 720 remains connected to the needle holder 702.

As shown in Figure 64B, during assembly of the device 700 the slider 714 is now pressed rearwards to butt up against the needle holder 702. The compression spring 720 is thus compressed. A catheter 726 is arranged to slide over the needle 704 and the body of the catheter 726B butts up against the slider 714, fitting over the boss 712 formed in the slider 714. A spring steel clip 730 is inserted into the slider 714. This clip consists of a hook 730H which engages over the rim of the catheter body 726B. The clip 730 connects the catheter to the slider 730 such that they move as one. A lid 732 clips onto the bottom of the slider 714 to contain the clip 730.

As can be seen in Figure 64C, a handle component 734 consists of a grip designed to fit a user's thumb and from which an arm 735 extends forwardly. The arm fits into a groove formed in the needle holder 702 so that the handle 734 can slide forwards and backwards in this groove. The underside of the arm 735 has a gear rack 735T formed in its face. An axle 740 is formed in the needle holder 702 between the upper 735T and lower 716 gear racks.

As seen in Figure 64D, a wheel 742 (shown with a cutaway) clips over the axle 740. The wheel 742 consists of a spur gear 743 which engages with the upper 735T and lower 716 racks. This gear 743 is connected to an outer rim 744. The rim 744 is formed so that it is shaped like a single gear tooth 744T at one end. This tooth 744T acts against a face formed on the moulding 708 of the probe 706. When the wheel 742 rotates anticlockwise (as viewed) the gear tooth 744T on the rim 744 pushes the probe 706 forwards relative to the needle 704.

Figure 64E shows the assembled device 700 ready for use. Grips A and B which are sized to be gripped by a user's thumb and finger are highlighted.

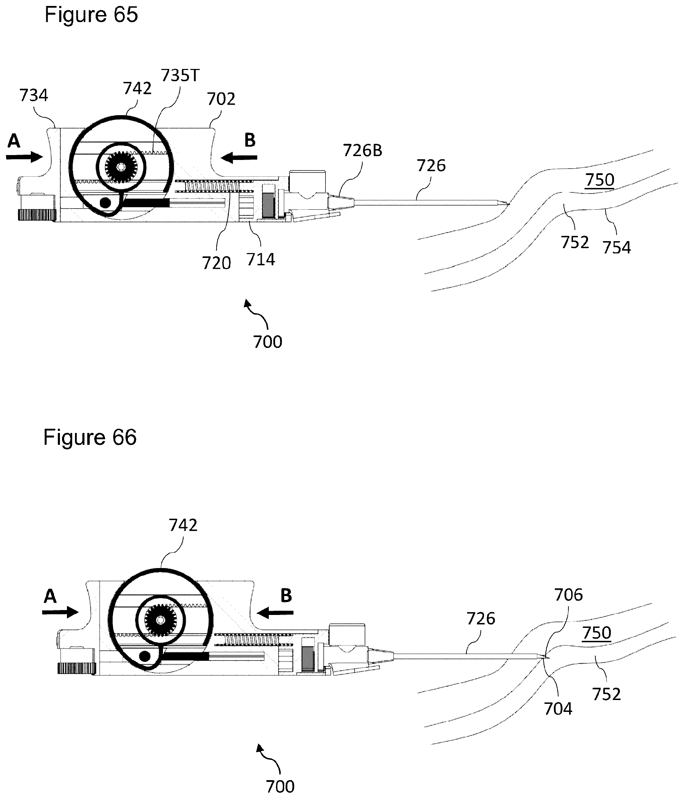

The sequence of operation of the device is now described with reference to the further drawings Figures 65 to 71. In the initial condition shown in Figure 65, the device is held in a "compressed" state by a finger of the user located in grip B squeezing against the thumb of the user located in grip A. Furthermore, the compression spring 720 is urging the slider 714 away from the needle holder 702. The lower rack of the slider 714 would also tend to rotate the wheel 742 anticlockwise (as viewed) which would push the upper rack 735T of the handle 734 rearwards relative to the needle holder 702. However, this is prevented by the light squeeze of the user.

In the "Advance through tissue' stage of operation shown in Figure 66, the device 700 is held in the "compressed" state as the user pushes the device forwards, forcing the needle 704, probe 706 and catheter 726 through the tissue 750 of a patient.

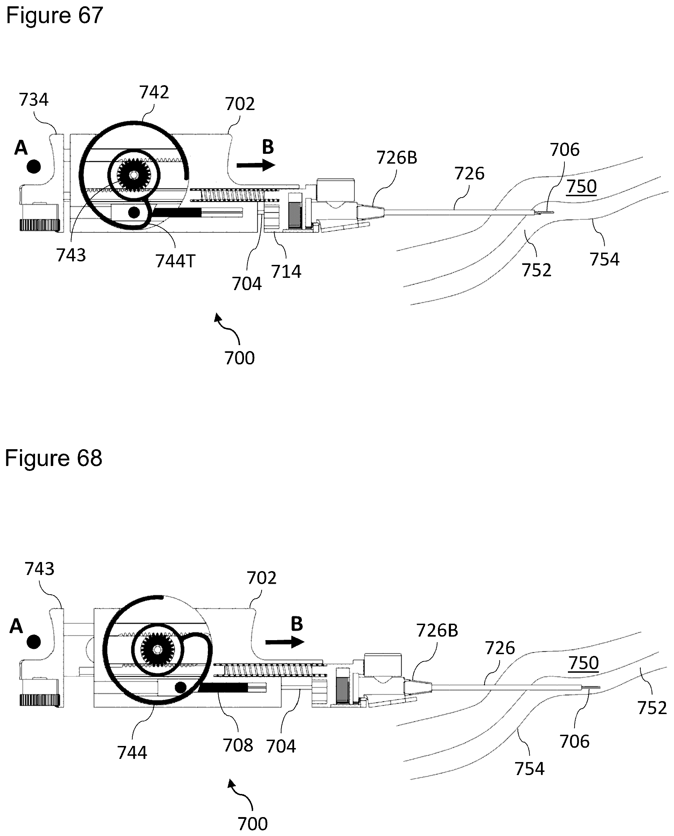

In the "Breakthrough stage" shown in Figure 67, the needle 702 breaks through into the lumen 752 of the vein 754, a "flash" of blood is observed - either in the flash chamber 711 or at the notches 704N in the distal end of the needle 704. On observing the flash, the user keeps their thumb at grip A stationary relative to the patient and gently relaxes the squeeze in the finger at grip B. This allows the needle holder 702 to move forward relative to the handle 734 and the slider 714 to move forward relative to the needle holder 702. Meanwhile, the wheel 742 rotates anticlockwise (as viewed) pushing the probe 706 forward relative to the needle holder 702. Because the effective radius of the gear tooth 744T on the wheel rim 744 is greater than the radius of the spur gear 743, the probe 706 advances faster than the catheter 726 while the needle 704 retracts relative to the catheter 726.

The "Advance into the vein" stage is shown in Figure 68. In this stage, the user's finger at B continues to relax away from their thumb at B. The needle holder 702 continues to be advanced relative to the stationary handle 734, and the catheter 726 advances further into the lumen 752 of vein 754. While the needle 704 has continued to retract relative to the catheter 726 as the gear rolls backwards along the lower rack 716, the probe over-moulding 708 is now running along a section of the rim 744 with constant radius and so is no longer advanced relative to the needle 704. This means the catheter 726 "catches up" with the probe 706 towards the end of the insertion.

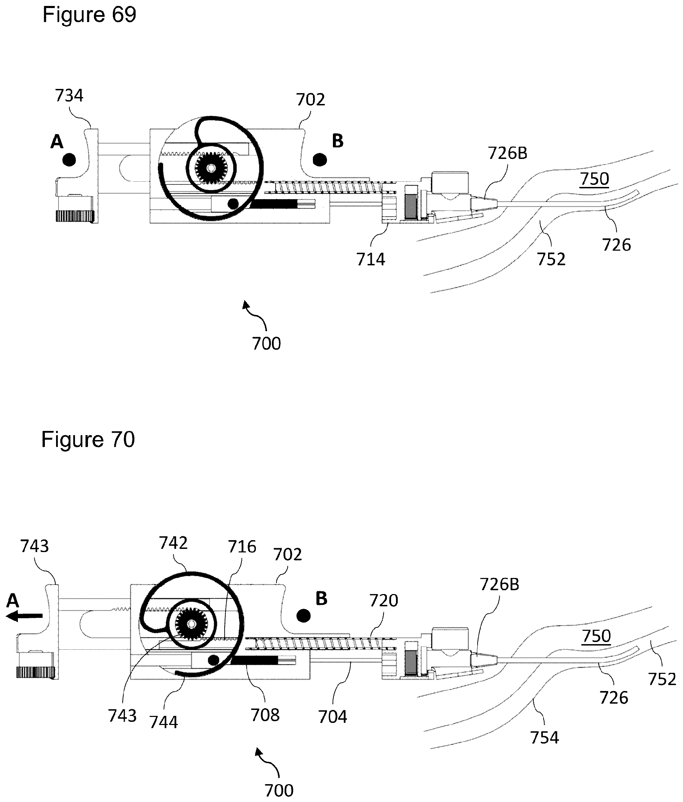

In the "End position" shown in Figure 69, the catheter 726 is fully advanced into the lumen 752 of vein 754. The tip of the catheter 726 has "overtaken" the end of the probe 706.

In the "Retract" stage shown in Figure 70, once the catheter 726 is fully inserted, the thumb at grip A is eased rearward which allows the wheel 742 to roll back further anticlockwise (as viewed). This allows the gear 743 to roll past the end of the lower rack 716.

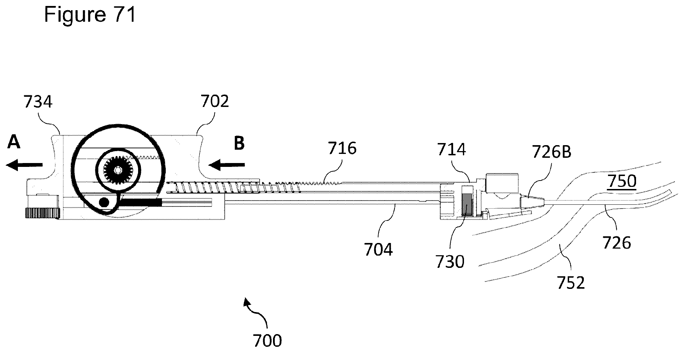

Finally, in the "Remove" stage shown in Figure 71, the catheter 726 and slider 714 are held stationary. Grips A and B can be simultaneously squeezed together and moved rearwards to retract needle 704. The needle 704 retracts from the catheter 726 until the needle tip is safely within the slider housing 714. Once the tip of the needle 704 has passed the spring steel clip 730, the clip slides across to prevent the needle tip moving forwards and at the same time releases the catheter 726 from the slider 714. The slider 714 is prevented from moving further forward than shown by a stop at the end of the groove in the needle holder 702 (not shown). The tip of the needle 704 is therefore safe within the slider 714 and the device 700 can now be removed from the thus inserted catheter 726 and disposed of safely.

A device in accordance with this embodiment may have several advantages. It can be used one-handed and requires fewer manipulations than a conventional device. Again, unlike some known devices, such as that described in US5,330,432, there is no trigger mechanism - which results in a smoother action on entering the vein. The relatively low part count and other features of the design may result in reduced production costs.

Whilst a number of embodiments have been described above, by way of example only, it will be appreciated by the skilled addressee that numerous modifications and variations can be made to those embodiments without departing from the scope of the invention defined by the appended claims. Numerous further embodiments within the scope of the invention defined by the appended claims and which are not based on the embodiments disclosed in this application may also be conceived by the skilled addressee.

## Claims

1. A device (10) for inserting a tube into a body cavity of a patient, the device (10) comprising:
a tube;
a probe (30);
a needle (14) configured to enter the body cavity, and to enable entry of the tube and the probe (30) into the body cavity; and
a drive mechanism configured to distally advance the needle (14), the tube and the probe (30) in the body cavity after entry of the probe (30) into the body cavity, with the probe (30) advancing distally beyond the needle (14).

2. The device (10) of claim 1, wherein the device (10) is configured such that, when the needle (14) is inserted through a region of body tissue (18) prior to entry into the body cavity, the tube and the probe (30) move with the needle (14), wherein, optionally:
the drive mechanism is configured to apply a biasing force to the probe (30) during insertion of the needle (14) through the region of body tissue (18), wherein the biasing force causes the probe (30) to advance distally beyond the needle (14) after entry of the probe (30) into the body cavity.

3. The device (10) of claim 1 or claim 2, wherein the device (10) is configured such that the probe (30) advances at a first rate causing distal advancement of the probe (30) relative to the tube after entry of the probe (30) into the body cavity, and subsequently advances at a second rate causing distal advancement of the tube relative to the probe (30).

4. The device (10) of any of claims 1 to 3, wherein the device (10) is configured such that the tube advances distally relative to the needle (14) after entry of the probe (30) into the body cavity.

5. The device of claim 4, wherein the device (10) is configured such that the tube advances distally beyond the probe (30) after distal advancement of the probe (30) beyond the needle (14).

6. The device of any of claims 1 to 5, wherein the drive mechanism is configured to retract the needle (14) and/or the probe (30) after insertion of the tube into the body cavity.

7. The device (10) of any of claims 1 to 6, wherein the tube, the probe (30) and the needle (14) are arranged concentrically.

8. The device (10) of any of claims 1 to 7, further comprising a support leg configured to abut a body surface of the patient during entry of the needle (14) into the body cavity, wherein the support leg is configured to drive the drive mechanism as the tube advances distally relative to the support leg.

9. The device (10) of any of claims 1 to 8, wherein the drive mechanism comprises a rotatable element coupled to each of the needle (14), the tube and the probe (30) via a respective mechanical linkage, and wherein the rate of distal advancement of each of the needle (14), the tube and the probe (30) is optionally determined by the distance between an end of its respective mechanical linkage that is coupled to the rotatable element and an axle (34) of the rotatable element.

10. The device (10) of any of claims 1 to 9, wherein the drive mechanism is operable to be driven in a first direction to distally advance the needle (14), the probe (30) and the tube.

11. The device (10) of claim 10, wherein the drive mechanism is operable to be driven in a second direction opposite to the first direction, to retract the probe (30), and wherein the drive mechanism is optionally operable to be driven in the second direction to retract the needle (14).

12. The device (10) of any of claims 1 to 11, further comprising a coupling element configured to couple the tube to the device (10) and to permit decoupling of the tube from the device (10) after insertion of the tube into the body cavity.

13. The device (10) of claim 12, wherein the coupling element is configured to decouple the tube from the device (10) after insertion of the tube into the body cavity, and/or wherein the coupling element is configured to cover a tip (14T) of the needle (14) after retraction of the needle (14) from the body cavity.

14. The device (10) according to any of claims 1 to 13, further comprising a support leg for contacting the patient's skin to maintain a spatial relationship between a body (506) of the device (10) and the patient.

15. The device (10) according to claim 8 or claim 14, wherein the support leg comprises a detachable component, wherein the support leg engages the tube in a fully deployed condition of the tube, and wherein the engaged support leg forms a wing or other element for fixing the tube to the patient.

## Patentansprüche

1. Vorrichtung (10) zum Einführen eines Schlauchs in eine Körperhöhle eines Patienten, wobei die Vorrichtung (10) Folgendes umfasst:
einen Schlauch;
eine Sonde (30);
eine Nadel (14), die dazu ausgelegt ist, in die Körperhöhle einzudringen und den Eintritt des Schlauchs und der Sonde (30) in die Körperhöhle zu ermöglichen; und
einen Antriebsmechanismus, der dazu ausgelegt ist, die Nadel (14), den Schlauch und die Sonde (30) nach dem Eintritt der Sonde (30) in die Körperhöhle distal vorwärts zu bewegen, wobei sich die Sonde (30) distal über die Nadel (14) hinaus vorwärts bewegt.

2. Vorrichtung (10) nach Anspruch 1, wobei die Vorrichtung (10) so ausgelegt ist, dass, wenn die Nadel (14) vor dem Eintritt in die Körperhöhle durch einen Bereich von Körpergewebe (18) eingeführt wird, sich der Schlauch und die Sonde (30) mit der Nadel (14) bewegen, wobei optional:
der Antriebsmechanismus dazu ausgelegt ist, während des Einführens der Nadel (14) durch den Bereich des Körpergewebes (18) eine Vorspannkraft auf die Sonde (30) auszuüben, wobei die Vorspannkraft bewirkt, dass sich die Sonde (30) nach dem Eintritt der Sonde (30) in die Körperhöhle distal über die Nadel (14) hinaus vorwärts bewegt.

3. Vorrichtung (10) nach Anspruch 1 oder Anspruch 2, wobei die Vorrichtung (10) so ausgelegt ist, dass sich die Sonde (30) mit einer ersten Geschwindigkeit vorwärts bewegt, die ein distales Vorschieben der Sonde (30) relativ zu dem Schlauch nach dem Eintritt der Sonde (30) in die Körperhöhle bewirkt, und sich anschließend mit einer zweiten Geschwindigkeit vorwärts bewegt, die ein distales Vorwärtsbewegen des Schlauchs relativ zu der Sonde (30) bewirkt.

4. Vorrichtung (10) nach einem der Ansprüche 1 bis 3, wobei die Vorrichtung (10) so ausgelegt ist, dass sich der Schlauch nach dem Eintritt der Sonde (30) in die Körperhöhle distal relativ zu der Nadel (14) vorwärts bewegt.

5. Vorrichtung nach Anspruch 4, wobei die Vorrichtung (10) so ausgelegt ist, dass sich der Schlauch nach dem distalen Vorwärtsbewegen der Sonde (30) über die Nadel (14) hinaus distal über die Sonde (30) hinaus vorwärts bewegt.

6. Vorrichtung nach einem der Ansprüche 1 bis 5, wobei der Antriebsmechanismus dazu ausgelegt ist, die Nadel (14) und/oder die Sonde (30) nach dem Einführen des Schlauchs in die Körperhöhle zurückzuziehen.

7. Vorrichtung (10) nach einem der Ansprüche 1 bis 6, wobei der Schlauch, die Sonde (30) und die Nadel (14) konzentrisch angeordnet sind.

8. Vorrichtung (10) nach einem der Ansprüche 1 bis 7, ferner umfassend ein Stützbein, das dazu ausgelegt ist, während des Eintritts der Nadel (14) in die Körperhöhle an einer Körperoberfläche des Patienten anzuliegen, wobei das Stützbein dazu ausgelegt ist, den Antriebsmechanismus anzutreiben, wenn sich der Schlauch distal relativ zu dem Stützbein vorwärts bewegt.

9. Vorrichtung (10) nach einem der Ansprüche 1 bis 8, wobei der Antriebsmechanismus ein drehbares Element umfasst, das über eine jeweilige mechanische Verbindung mit der Nadel (14), dem Schlauch und der Sonde (30) gekoppelt ist, und wobei die Rate des distalen Vorwärtsbewegens sowohl der Nadel (14) als auch des Schlauchs und der Sonde (30) optional durch den Abstand zwischen einem Ende ihrer jeweiligen mechanischen Verbindung, die mit dem drehbaren Element gekoppelt ist, und einer Achse (34) des drehbaren Elements bestimmt wird.

10. Vorrichtung (10) nach einem der Ansprüche 1 bis 9, wobei der Antriebsmechanismus betreibbar ist, um in einer ersten Richtung angetrieben zu werden, um die Nadel (14), die Sonde (30) und den Schlauch distal vorwärts zu bewegen.

11. Vorrichtung (10) nach Anspruch 10, wobei der Antriebsmechanismus betreibbar ist, um in eine zweite Richtung entgegengesetzt zu der ersten Richtung angetrieben zu werden, um die Sonde (30) zurückzuziehen, und wobei der Antriebsmechanismus optional betreibbar ist, um in die zweite Richtung angetrieben zu werden, um die Nadel (14) zurückzuziehen.

12. Vorrichtung (10) nach einem der Ansprüche 1 bis 11, die ferner ein Kopplungselement umfasst, das dazu ausgelegt ist, den Schlauch mit der Vorrichtung (10) zu koppeln und das Entkoppeln des Schlauchs von der Vorrichtung (10) nach dem Einführen des Schlauchs in die Körperhöhle zu ermöglichen.

13. Vorrichtung (10) nach Anspruch 12, wobei das Kopplungselement dazu ausgelegt ist, den Schlauch von der Vorrichtung (10) nach dem Einführen des Schlauchs in die Körperhöhle zu entkoppeln, und/oder wobei das Kopplungselement dazu ausgelegt ist, eine Spitze (14T) der Nadel (14) nach dem Zurückziehen der Nadel (14) aus der Körperhöhle abzudecken.

14. Vorrichtung (10) nach einem der Ansprüche 1 bis 13, ferner umfassend ein Stützbein zum Inkontaktkommen mit der Haut des Patienten, um eine räumliche Beziehung zwischen einem Körper (506) der Vorrichtung (10) und dem Patienten aufrechtzuerhalten.

15. Vorrichtung (10) nach Anspruch 8 oder Anspruch 14, wobei das Stützbein eine abnehmbare Komponente umfasst, wobei das Stützbein in einem vollständig ausgefahrenen Zustand des Schlauchs in Eingriff mit dem Schlauch kommt und wobei das in Eingriff stehende Stützbein einen Flügel oder ein anderes Element zum Fixieren des Schlauchs an dem Patienten bildet.

## Revendications

1. Dispositif (10) d'insertion d'un tube dans une cavité corporelle d'un patient, le dispositif (10) comprenant :
un tube ;
une sonde (30) ;
une aiguille (14) configurée pour entrer dans la cavité corporelle, et pour permettre l'entrée du tube et de la sonde (30) dans la cavité corporelle ; et
un mécanisme d'entraînement configuré pour faire avancer l'aiguille (14), le tube et la sonde (30) de façon distale dans la cavité corporelle après l'entrée de la sonde (30) dans la cavité corporelle, la sonde (30) avançant de façon distale au-delà de l'aiguille (14).

2. Dispositif (10) selon la revendication 1, le dispositif (10) étant configuré de telle sorte que, lorsque l'aiguille (14) est insérée à travers une région de tissu corporel (18) avant l'entrée dans la cavité corporelle, le tube et la sonde (30) se déplacent avec l'aiguille (14), facultativement :
le mécanisme d'entraînement étant configuré pour appliquer une force de sollicitation à la sonde (30) pendant l'insertion de l'aiguille (14) à travers la région de tissu corporel (18), la force de sollicitation faisant avancer la sonde (30) de façon distale au-delà de l'aiguille (14) après l'entrée de la sonde (30) dans la cavité corporelle.

3. Dispositif (10) selon la revendication 1 ou la revendication 2, le dispositif (10) étant configuré de telle sorte que la sonde (30) avance à une première vitesse provoquant l'avancement distal de la sonde (30) par rapport au tube après l'entrée de la sonde (30) dans la cavité corporelle, et avance ensuite à une seconde vitesse provoquant l'avancement distal du tube par rapport à la sonde (30).

4. Dispositif (10) selon l'une quelconque des revendications 1 à 3, le dispositif (10) étant configuré de telle sorte que le tube avance de façon distale par rapport à l'aiguille (14) après l'entrée de la sonde (30) dans la cavité corporelle.

5. Dispositif selon la revendication 4, le dispositif (10) étant configuré de telle sorte que le tube avance de façon distale au-delà de la sonde (30) après l'avancement distal de la sonde (30) au-delà de l'aiguille (14).

6. Dispositif selon l'une quelconque des revendications 1 à 5, le mécanisme d'entraînement étant configuré pour rétracter l'aiguille (14) et/ou la sonde (30) après insertion du tube dans la cavité corporelle.

7. Dispositif (10) selon l'une quelconque des revendications 1 à 6, le tube, la sonde (30) et l'aiguille (14) étant agencés concentriquement.

8. Dispositif (10) selon l'une quelconque des revendications 1 à 7, comprenant en outre un pied de support configuré pour buter contre une surface corporelle du patient pendant l'entrée de l'aiguille (14) dans la cavité corporelle, le pied de support étant configuré pour entraîner le mécanisme d'entraînement lorsque le tube avance de façon distale par rapport au pied de support.

9. Dispositif (10) selon l'une quelconque des revendications 1 à 8, le mécanisme d'entraînement comprenant un élément rotatif couplé respectivement à l'aiguille (14), au tube et à la sonde (30) par l'intermédiaire d'une liaison mécanique respective, et la vitesse d'avancement distal respectivement de l'aiguille (14), du tube et de la sonde (30) étant facultativement déterminée par la distance entre, une extrémité de sa liaison mécanique respective qui est couplée à l'élément rotatif, et un axe (34) de l'élément rotatif.

10. Dispositif (10) selon l'une quelconque des revendications 1 à 9, le mécanisme d'entraînement pouvant être actionné pour être entraîné dans une première direction pour faire avancer de façon distale l'aiguille (14), la sonde (30) et le tube.

11. Dispositif (10) selon la revendication 10, le mécanisme d'entraînement pouvant être actionné pour être entraîné dans une seconde direction opposée à la première direction, pour rétracter la sonde (30), et le mécanisme d'entraînement pouvant facultativement être actionné pour être entraîné dans la seconde direction pour rétracter l'aiguille (14).

12. Dispositif (10) selon l'une quelconque des revendications 1 à 11, comprenant en outre un élément de couplage configuré pour coupler le tube au dispositif (10) et pour permettre de découpler le tube du dispositif (10) après insertion du tube dans la cavité corporelle.

13. Dispositif (10) selon la revendication 12, l'élément de couplage étant configuré pour découpler le tube du dispositif (10) après insertion du tube dans la cavité corporelle, et/ou l'élément de couplage étant configuré pour recouvrir une pointe (14T) de l'aiguille (14) après rétraction de l'aiguille (14) hors de la cavité corporelle.

14. Dispositif (10) selon l'une quelconque des revendications 1 à 13, comprenant en outre un pied de support pour entrer en contact avec la peau du patient afin de maintenir une relation spatiale entre un corps (506) du dispositif (10) et le patient.

15. Dispositif (10) selon la revendication 8 ou la revendication 14, le pied de support comprenant un composant détachable, le pied de support venant en prise avec le tube dans un état complètement déployé du tube, et le pied de support en prise formant une ailette ou un autre élément pour fixer le tube au patient.
